# EUROPEAN PATENT APPLICATION

(11) **EP 2 508 202 A1**
(43) Date of publication of application: **10.10.2012**
(21) Application number: 11382103.7
(22) Date of filing: 07.04.2011
(51) Int. Cl.: A61K 39/21, C07K 14/16, C12N 7/04, C12N 15/867

(54) **Non-replicative virions of human immunodeficiency virus and therapeutic applications thereof**

(71) Applicant: Fundació Clínic per a la Recerca Biomèdica, 08036 Barcelona (ES); Institut De Investigacions Biomediques August PI I Sunyer, 08036 Barcelona (ES); Instituto de Salud Carlos III, 28029 Madrid (ES)
(72) Inventor: Sánchez-Palomino, Sonsoles, E-08036, Barcelona (ES); Plana Prades, Monserrat, E-08036, Barcelona (ES); Alcami Pertejo, José, E-28029, Madrid (ES)
(74) Representative: ABG Patentes, S.L.

(57) **Abstract**

The present invention relates to the development of viral vectors comprising a human immunodeficiency virus (HIV) genome and non-replicative virions comprising said viral vectors. Said viral vectors and non-replicative virions are useful for use in medicine, specifically for use in generating vaccines and more specifically for treating AIDS. The present invention further relates to a method for obtaining a customized vaccine.

SEQUENCE LISTING

<110>  FUNDACIÓ CLINIC PER A LA RECERCA BIOMÈDICA
       INSTITUT D'INVESTIGACIONS BIOMÈDIQUES AUGUST PI I SUNYER
       INSTITUTO DE SALUD CARLOS III

<120>  Non-replicative virions of human immunodeficiency virus and its
       therapeutic uses

<130>  P5745EP00

<160>  41

<170>  PatentIn version 3.5

<210>  1
<211>  14825
<212>  DNA
<213>  Human immunodeficiency virus type 1

<400>  1
tggaagggct aatttggtcc caaaaaagac aagagatcct tgatctgtgg atctaccaca      60

cacaaggcta cttccctgat tggcagaact acacaccagg gccagggatc agatatccac     120

tgacctttgg atggtgcttc aagttagtac cagttgaacc agagcaagta gaagaggcca     180

atgaaggaga gaacaacagc ttgttacacc ctatgagcca gcatgggatg gaggacccgg     240

agggagaagt attagtgtgg aagtttgaca gcctcctagc atttcgtcac atggcccgag     300

agctgcatcc ggagtactac aaagactgct gacatcgagc tttctacaag ggactttccg     360

ctggggactt tccagggagg tgtggcctgg gcgggactgg ggagtggcga gccctcagat     420

gctacatata agcagctgct ttttgcctgt actgggtctc tctggttaga ccagatctga     480

gcctgggagc tctctggcta actagggaac ccactgctta agcctcaata aagcttgcct     540

tgagtgctca aagtagtgtg tgcccgtctg ttgtgtgact ctggtaacta gagatccctc     600

agaccctttt agtcagtgtg gaaaatctct agcagtggcg cccgaacagg gacttgaaag     660

cgaaagtaaa gccagaggag atctctcgac gcaggactcg gcttgctgaa gcgcgcacgg     720

caagaggcga ggggcggcga ctggtgagta cgccaaaaat tttgactagc ggaggctaga     780

aggagagaga tgggtgcgag agcgtcggta ttaagcgggg gagaattaga taaatgggaa     840

aaaattcggt taaggccagg gggaaagaaa caatataaac taaaacatat agtatgggca     900

agcagggagc tagaacgatt cgcagttaat cctggccttt tagagacatc agaaggctgt     960

agacaaatac tgggacagct acaaccatcc cttcagacag gatcagaaga acttagatca    1020

ttatataata caatagcagt cctctattgt gtgcatcaaa ggatagatgt aaaagacacc    1080

aaggaagcct tagataagat agaggaagag caaaacaaaa gtaagaaaaa ggcacagcaa 1140

gcagcagctg acacaggaaa caacagccag gtcagccaaa attaccctat agtgcagaac 1200

ctccaggggc aaatggtaca tcaggccata tcacctagaa ctttaaatgc atgggtaaaa 1260

gtagtagaag agaaggcttt cagcccagaa gtaataccca tgttttcagc attatcagaa 1320

ggagccaccc cacaagattt aaataccatg ctaaacacag tggggggaca tcaagcagcc 1380

atgcaaatgt taaaagagac catcaatgag gaagctgcag aatgggatag attgcatcca 1440

gtgcatgcag ggcctattgc accaggccag atgagagaac caaggggaag tgacatagca 1500

ggaactacta gtacccttca ggaacaaata ggatggatga cacataatcc acctatccca 1560

gtaggagaaa tctataaaag atggataatc ctgggattaa ataaaatagt aagaatgtat 1620

agccctacca gcattctgga cataagacaa ggaccaaagg aaccctttag agactatgta 1680

gaccgattct ataaaactct aagagccgag caagcttcac aagaggtaaa aaattggatg 1740

acagaaacct tgttggtcca aaatgcgaac ccagattgta agactatttt aaaagcattg 1800

ggaccaggag cgacactaga agaaatgatg acagcatgtc agggagtggg gggacccggc 1860

cataaagcaa gagttttggc tgaagcaatg agccaagtaa caaatccagc taccataatg 1920

atacagaaag gcaattttag gaaccaaaga aagactgtta agtgtttcaa ttgtggcaaa 1980

gaagggcaca tagccaaaaa ttgcagggcc cctaggaaaa agggctgttg gaaatgtgga 2040

aaggaaggac accaaatgaa agattgtact gagagacagg ctaatttttt agggaagatc 2100

tggccttccc acaagggaag gccagggaat tttcttcaga gcagaccaga gccaacagcc 2160

ccaccagaag agagcttcag gtttggggaa gagacaacaa ctccctctca gaagcaggag 2220

ccgatagaca aggaactgta tcctttagct tccctcagat cactctttgg cagcgacccc 2280

tcgtcacaat aaagataggg gggcaattaa aggaagctct attagataca ggagcagatg 2340

atacagtatt agaagaaatg aatttgccag gaagatggaa accaaaaatg atagggggaa 2400

ttggaggttt tatcaaagta agacagtatg atcagatact catagaaatc tgcggacata 2460

aagctatagg tacagtatta gtaggaccta cacctgtcaa cataattgga agaaatctgt 2520

tgactcagat tggctgcact ttaaattttc ccattagtcc tattgagact gtaccagtaa 2580

aattaaagcc aggaatggat ggcccaaaag ttaaacaatg gccattgaca gaagaaaaaa 2640

taaaagcatt agtagaaatt tgtacagaaa tggaaaagga aggaaaaatt tcaaaaattg 2700

ggcctgaaaa tccatacaat actccagtat ttgccataaa gaaaaaagac agtactaaat 2760

ggagaaaatt agtagatttc agagaactta ataagagaac tcaagatttc tgggaagttc 2820

aattaggaat accacatcct gcagggttaa aacagaaaaa atcagtaaca gtactggatg 2880

tgggcgatgc atatttttca gttcccttag ataaagactt caggaagtat actgcattta 2940

ccatacctag tataaacaat gagacaccag ggattagata tcagtacaat gtgcttccac 3000

agggatggaa aggatcacca gcaatattcc agtgtagcat gacaaaaatc ttagagcctt 3060

ttagaaaaca aaatccagac atagtcatct atcaatacat ggatgatttg tatgtaggat 3120

ctgacttaga aatagggcag catagaacaa aaatagagga actgagacaa catctgttga 3180

ggtggggatt taccacacca gacaaaaaac atcagaaaga acctccattc ctttggatgg 3240

gttatgaact ccatcctgat aaatggacag tacagcctat agtgctgcca gaaaaggaca 3300

gctggactgt caatgacata cagaaattag tgggaaaatt gaattgggca agtcagattt 3360

atgcagggat taaagtaagg caattatgta aacttcttag gggaaccaaa gcactaacag 3420

aagtagtacc actaacagaa gaagcagagc tagaactggc agaaaacagg gagattctaa 3480

aagaaccggt acatggagtg tattatgacc catcaaaaga cttaatagca gaaatacaga 3540

agcaggggca aggccaatgg acatatcaaa tttatcaaga gccatttaaa aatctgaaaa 3600

caggaaagta tgcaagaatg aagggtgccc acactaatga tgtgaaacaa ttaacagagg 3660

cagtacaaaa aatagccaca gaaagcatag taatatgggg aaagactcct aaatttaaat 3720

tacccataca aaaggaaaca tgggaagcat ggtggacaga gtattggcaa gccacctgga 3780

ttcctgagtg ggagtttgtc aatacccctc ccttagtgaa gttatggtac cagttagaga 3840

aagaacccat aataggagca gaaactttct atgtagatgg ggcagccaat agggaaacta 3900

aattaggaaa agcaggatat gtaactgaca gaggaagaca aaaagttgtc cccctaacgg 3960

acacaacaaa tcagaagact gagttacaag caattcatct agctttgcag gattcgggat 4020

tagaagtaaa catagtgaca gactcacaat atgcattggg aatcattcaa gcacaaccag 4080

ataagagtga atcagagtta gtcagtcaaa taatagagca gttaataaaa aaggaaaaag 4140

tctacctggc atgggtacca gcacacaaag gaattggagg aaatgaacaa gtagataaat 4200

tggtcagtgc tggaatcagg aaagtactat ttttagatgg aatagataag gcccaagaag 4260

aacatgagaa atatcacagt aattggagag caatggctag tgattttaac ctaccacctg 4320

tagtagcaaa agaaatagta gccagctgtg ataaatgtca gctaaaaggg gaagccatgc 4380

atggacaagt agactgtagc ccaggaatat ggcagctaga ttgtacacat ttagaaggaa 4440

aagttatctt ggtagcagtt catgtagcca gtggatatat agaagcagaa gtaattccag 4500

cagagacagg gcaagaaaca gcatacttcc tcttaaaatt agcaggaaga tggccagtaa 4560

aaacagtaca tacagacaat ggcagcaatt tcaccagtac tacagttaag gccgcctgtt 4620

ggtgggcggg gatcaagcag gaatttggca ttccctacaa tccccaaagt caaggagtaa 4680

tagaatctat gaataaagaa ttaaagaaaa ttataggaca ggtaagagat caggctgaac 4740

atcttaagac agcagtacaa atggcagtat tcatccacaa ttttaaaaga aaagggggga 4800

ttggggggta cagtgcaggg gaaagaatag tagacataat agcaacagac atacaaacta 4860

aagaattaca aaaacaaatt acaaaaattc aaaattttcg ggtttattac agggacagca 4920

gagatccagt ttggaaagga ccagcaaagc tcctctggaa aggtgaaggg gcagtagtaa 4980

tacaagataa tagtgacata aaagtagtgc caagaagaaa agcaaagatc atcagggatt 5040

atggaaaaca gatggcaggt gatgattgtg tggcaagtag acaggatgag gattaacaca 5100

tggaaaagat tagtaaaaca ccatatgtat atttcaagga aagctaagga ctggttttat 5160

agacatcact atgaaagtac taatccaaaa ataagttcag aagtacacat cccactaggg 5220

gatgctaaat tagtaataac aacatattgg ggtctgcata caggagaaag agactggcat 5280

ttgggtcagg gagtctccat agaatggagg aaaaagagat atagcacaca agtagaccct 5340

gacctagcag accaactaat tcatctgcac tattttgatt gtttttcaga atctgctata 5400

agaaatacca tattaggacg tatagttagt cctaggtgtg aatatcaagc aggacataac 5460

aaggtaggat ctctacagta cttggcacta gcagcattaa taaaaccaaa acagataaag 5520

ccacctttgc ctagtgttag gaaactgaca gaggacagat ggaacaagcc ccagaagacc 5580

aagggccaca gagggagcca tacaatgaat ggacactaga gcttttagag gaacttaaga 5640

gtgaagctgt tagacatttt cctaggatat ggctccataa cttaggacaa catatctatg 5700

aaacttacgg ggatacttgg gcaggagtgg aagccataat aagaattctg caacaactgc 5760

tgtttatcca tttcagaatt gggtgtcgac atagcagaat aggcgttact cgacagagga 5820

gagcaagaaa tggagccagt agatcctaga ctagagccct ggaagcatcc aggaagtcag 5880

cctaaaactg cttgtaccaa ttgctattgt aaaaagtgtt gctttcattg ccaagtttgt 5940

ttcatgacaa aagccttagg catctcctat ggcaggaaga agcggagaca gcgacgaaga 6000

gctcatcaga acagtcagac tcatcaagct tctctatcaa agcagtaagt agtacatgta 6060

atgcaaccta taatagtagc aatagtagca ttagtagtag caataataat agcaatagtt 6120

gtgtggtcca tagtaatcat agaatatagg aaaatattaa gacaaagaaa aatagacagg 6180

ttaattgata gactaataga aagagcagaa gacagtggca atgagagtga aggagaagta 6240

tcagcacttg tggagatggg ggtggaaatg gggcaccatg ctccttggga tattgatgat 6300

ctgtagtgct acagaaaaat tgtgggtcac agtctattat ggggtacctg tgtggaagga 6360

agcaaccacc actctatttt gtgcatcaga tgctaaagca tatgatacag aggtacataa 6420

tgtttgggcc acacatgcct gtgtacccac agaccccaac ccacaagaag tagtattggt 6480

aaatgtgaca gaaaatttta acatgtggaa aaatgacatg gtagaacaga tgcatgagga 6540

tataatcagt ttatgggatc aaagcctaaa gccatgtgta aaattaaccc cactctgtgt 6600

tagtttaaag tgcactgatt tgaagaatga tactaatacc aatagtagta gcgggagaat 6660

gataatggag aaaggagaga taaaaaactg ctctttcaat atcagcacaa gcataagaga 6720

taaggtgcag aaagaatatg cattctttta taaacttgat atagtaccaa tagataatac 6780

cagctatagg ttgataagtt gtaacacctc agtcattaca caggcctgtc caaaggtatc 6840

ctttgagcca attcccatac attattgtgc cccggctggt tttgcgattc taaaatgtaa 6900

taataagacg ttcaatggaa caggaccatg tacaaatgtc agcacagtac aatgtacaca 6960

tggaatcagg ccagtagtat caactcaact gctgttaaat ggcagtctag cagaagaaga 7020

tgtagtaatt agatctgcca atttcacaga caatgctaaa accataatag tacagctgaa 7080

cacatctgta gaaattaatt gtacaagacc caacaacaat acaagaaaaa gtatccgtat 7140

ccagagggga ccagggagag catttgttac aataggaaaa ataggaaata tgagacaagc 7200

acattgtaac attagtagag caaaatggaa tgccacttta aaacagatag ctagcaaatt 7260

aagagaacaa tttggaaata ataaaacaat aatctttaag caatcctcag gaggggaccc 7320

agaaattgta acgcacagtt ttaattgtgg aggggaattt ttctactgta attcaacaca 7380

actgtttaat agtacttggt ttaatagtac ttggagtact gaagggtcaa ataacactga 7440

aggaagtgac acaatcacac tcccatgcag aataaaacaa tttataaaca tgtggcagga 7500

agtaggaaaa gcaatgtatg cccctcccat cagtggacaa attagatgtt catcaaatat 7560

tactgggctg ctattaacaa gagatggtgg taataacaac aatgggtccg agatcttcag 7620

acctggagga ggcgatatga gggacaattg gagaagtgaa ttatataaat ataaagtagt 7680

aaaaattgaa ccattaggag tagcacccac caaggcaaag agaagagtgg tgcagagaga 7740

aaaaagagca gtgggaatag gagctttgtt ccttgggttc ttgggagcag caggaagcac 7800

tatgggcgca gcgtcaatga cgctgacggt acaggccaga caattattgt ctgatatagt 7860

gcagcagcag aacaatttgc tgagggctat tgaggcgcaa cagcatctgt tgcaactcac 7920

agtctggggc atcaaacagc tccaggcaag aatcctggct gtggaaagat acctaaagga 7980

tcaacagctc ctggggattt ggggttgctc tggaaaactc atttgcacca ctgctgtgcc 8040

ttggaatgct agttggagta ataaatctct ggaacagatt tggaataaca tgacctggat 8100

ggagtgggac agagaaatta acaattacac aagcttaata cactccttaa ttgaagaatc 8160

gcaaaaccag caagaaaaga atgaacaaga attattggaa ttagataaat gggcaagttt 8220

gtggaattgg tttaacataa caaattggct gtggtatata aaattattca taatgatagt 8280

aggaggcttg gtaggtttaa gaatagtttt tgctgtactt tctatagtga atagagttag 8340

gcagggatat tcaccattat cgtttcagac ccacctccca atcccgaggg gacccgacag 8400

gcccgaagga atagaagaag aaggtggaga gagagacaga gacagatcca ttcgattagt 8460

gaacggatcc ttagcactta tctgggacga tctgcggagc ctgtgcctct tcagctacca 8520

ccgcttgaga gacttactct tgattgtaac gaggattgtg gaacttctgg gacgcagggg 8580

gtgggaagcc ctcaaatatt ggtggaatct cctacagtat tggagtcagg aactaaagaa 8640

tagtgctgtt aacttgctca atgccacagc catagcagta gctgagggga cagatagggt 8700

tatagaagta ttacaagcag cttatagagc tattcgccac atacctagaa gaataagaca 8760

gggcttggaa aggattttgc tataagatgg gtggcaagtg gtcaaaaagt agtgtgattg 8820

gatggcctgc tgtaaggaaa agaatgagac gagctgagcc agcagcagat ggggtgggag 8880

cagtatctcg agacctagaa aaacatggag caatcacaag tagcaataca gcagctaaca 8940

atgctgcttg tgcctggcta gaagcacaag aggaggaaga ggtgggtttt ccagtcacac 9000

ctcaggtacc tttaagacca atgacttaca aggcagctgt agatcttagc cactttttaa 9060

aagaaaaggg gggactggaa gggctaattc actcccaaag aagacaagat atccttgatc 9120

tgtggatcta ccacacacaa ggctacttcc ctgattggca gaactacaca ccagggccag 9180

gggtcagata tccactgacc tttggatggt gctacaagct agtaccagtt gagccagata 9240

aggtagaaga ggccaataaa ggagagaaca ccagcttgtt acaccctgtg agcctgcatg 9300

gaatggatga ccctgagaga gaagtgttag agtggaggtt tgacagccgc ctagcatttc 9360

atcacgtggc ccgagagctg catccggagt acttcaagaa ctgctgacat cgagcttgct 9420

acaagggact ttccgctggg gactttccag ggaggcgtgg cctgggcggg actggggagt 9480

ggcgagccct cagatgctgc atataagcag ctgctttttg cctgtactgg gtctctctgg 9540

ttagaccaga tctgagcctg ggagctctct ggctaactag ggaacccact gcttaagcct 9600

caataaagct tgccttgagt gcttcaagta gtgtgtgccc gtctgttgtg tgactctggt 9660

aactagagat ccctcagacc cttttagtca gtgtggaaaa tctctagcac ccaggaggta 9720

gaggttgcag tgagccaaga tcgcgccact gcattccagc ctgggcaaga aaacaagact 9780

gtctaaaata ataataataa gttaagggta ttaaatatat ttatacatgg aggtcataaa 9840

aatatatata tttgggctgg gcgcagtggc tcacacctgc gcccggccct ttgggaggcc 9900

gaggcaggtg gatcacctga gtttgggagt tccagaccag cctgaccaac atggagaaac 9960

cccttctctg tgtattttta gtagatttta ttttatgtgt attttattca caggtatttc 10020

tggaaaactg aaactgtttt tcctctactc tgataccaca agaatcatca gcacagagga 10080

agacttctgt gatcaaatgt ggtgggagag ggaggttttc accagcacat gagcagtcag 10140

ttctgccgca gactcggcgg gtgtccttcg gttcagttcc aacaccgcct gcctggagag 10200

aggtcagacc acagggtgag ggctcagtcc ccaagacata aacacccaag acataaacac 10260

ccaacaggtc caccccgcct gctgcccagg cagagccgat tcaccaagac gggaattagg 10320

atagagaaag agtaagtcac acagagccgg ctgtgcggga gaacggagtt ctattatgac 10380

tcaaatcagt ctccccaagc attcggggat cagagttttt aaggataact tagtgtgtag 10440

ggggccagtg agttggagat gaaagcgtag ggagtcgaag gtgtcctttt gcgccgagtc 10500

agttcctggg tgggggccac aagatcggat gagccagttt atcaatccgg gggtgccagc 10560

tgatccatgg agtgcagggt ctgcaaaata tctcaagcac tgattgatct taggttttac 10620

aatagtgatg ttaccccagg aacaatttgg ggaaggtcag aatcttgtag cctgtagctg 10680

catgactcct aaaccataat ttcttttttg tttttttttt tttatttttg agacagggtc 10740

tcactctgtc acctaggctg gagtgcagtg gtgcaatcac agctcactgc agcctcaacg 10800

tcgtaagctc aagcgatcct cccacctcag cctgcctggt agctgagact acaagcgacg 10860

ccccagttaa tttttgtatt tttggtagag gcagcgtttt gccgtgtggc cctggctggt 10920

ctcgaactcc tgggctcaag tgatccagcc tcagcctccc aaagtgctgg gacaaccggg 10980

gccagtcact gcacctggcc ctaaaccata atttctaatc ttttggctaa tttgttagtc 11040

ctacaaaggc agtctagtcc ccaggcaaaa agggggtttg tttcgggaaa gggctgttac 11100

tgtctttgtt tcaaactata aactaagttc ctcctaaact tagttcggcc tacacccagg 11160

aatgaacaag gagagcttgg aggttagaag cacgatggaa ttggttaggt cagatctctt 11220

tcactgtctg agttataatt ttgcaatggt ggttcaaaga ctgcccgctt ctgacaccag 11280

tcgctgcatt aatgaatcgg ccaacgcgcg gggagaggcg gtttgcgtat tgggcgctct 11340

tccgcttcct cgctcactga ctcgctgcgc tcggtcgttc ggctgcggcg agcggtatca 11400

gctcactcaa aggcggtaat acggttatcc acagaatcag gggataacgc aggaaagaac 11460

atgtgagcaa aaggccagca aaaggccagg aaccgtaaaa aggccgcgtt gctggcgttt 11520

ttccataggc tccgcccccc tgacgagcat cacaaaaatc gacgctcaag tcagaggtgg 11580

cgaaacccga caggactata aagataccag gcgtttcccc ctggaagctc cctcgtgcgc 11640

tctcctgttc cgaccctgcc gcttaccgga tacctgtccg cctttctccc ttcgggaagc 11700

gtggcgcttt ctcatagctc acgctgtagg tatctcagtt cggtgtaggt cgttcgctcc 11760

aagctgggct gtgtgcacga accccccgtt cagcccgacc gctgcgcctt atccggtaac 11820

tatcgtcttg agtccaaccc ggtaagacac gacttatcgc cactggcagc agccactggt 11880

aacaggatta gcagagcgag gtatgtaggc ggtgctacag agttcttgaa gtggtggcct 11940

aactacggct acactagaag aacagtattt ggtatctgcg ctctgctgaa gccagttacc 12000

ttcggaaaaa gagttggtag ctcttgatcc ggcaaacaaa ccaccgctgg tagcggtggt 12060

ttttttgttt gcaagcagca gattacgcgc agaaaaaaag gatctcaaga agatcctttg 12120

atcttttcta cggggtctga cgctcagtgg aacgaaaact cacgttaagg gattttggtc 12180

atgagattat caaaaaggat cttcacctag atccttttaa attaaaaatg aagttttaaa 12240

tcaatctaaa gtatatatga gtaaacttgg tctgacagtt accaatgctt aatcagtgag 12300

gcacctatct cagcgatctg tctatttcgt tcatccatag ttgcctgact ccccgtcgtg 12360

tagataacta cgatacggga gggcttacca tctggcccca gtgctgcaat gataccgcga 12420

gacccacgct caccggctcc agatttatca gcaataaacc agccagccgg aagggccgag 12480

cgcagaagtg gtcctgcaac tttatccgcc tccatccagt ctattaattg ttgccgggaa 12540

gctagagtaa gtagttcgcc agttaatagt ttgcgcaacg ttgttgccat tgctacaggc 12600

atcgtggtgt cacgctcgtc gtttggtatg gcttcattca gctccggttc ccaacgatca 12660

aggcgagtta catgatcccc catgttgtgc aaaaaagcgg ttagctcctt cggtcctccg 12720

atcgttgtca gaagtaagtt ggccgcagtg ttatcactca tggttatggc agcactgcat 12780

aattctctta ctgtcatgcc atccgtaaga tgcttttctg tgactggtga gtactcaacc 12840

aagtcattct gagaatagtg tatgcggcga ccgagttgct cttgcccggc gtcaatacgg 12900

gataataccg cgccacatag cagaacttta aaagtgctca tcattggaaa acgttcttcg 12960

gggcgaaaac tctcaaggat cttaccgctg ttgagatcca gttcgatgta acccactcgt 13020

gcacccaact gatcttcagc atcttttact ttcaccagcg tttctgggtg agcaaaaaca 13080

ggaaggcaaa atgccgcaaa aaagggaata agggcgacac ggaaatgttg aatactcata 13140

ctcttccttt ttcaatatta ttgaagcatt tatcagggtt attgtctcat gagcggatac 13200

atatttgaat gtatttagaa aaataaacaa ataggggttc cgcgcacatt tccccgaaaa 13260

gtgccacctg acgtctaaga aaccattatt atcatgacat taacctataa aaataggcgt 13320

atcacgaggc cctttcgtct cgcgcgtttc ggtgatgacg gtgaaaacct ctgacacatg 13380

cagctcccgg agacggtcac agcttgtctg taagcggatg ccgggagcag acaagcccgt 13440

cagggcgcgt cagcgggtgt tggcgggtgt cggggctggc ttaactatgc ggcatcagag 13500

cagattgtac tgagagtgca ccatatgcgg tgtgaaatac cgcacagatg cgtaaggaga 13560

aaataccgca tcaggcgcca ttcgccattc aggctgcgca actgttggga agggcgatcg 13620

gtgcgggcct cttcgctatt acgccagggg aggcagagat tgcagtaagc tgagatcgca 13680

gcactgcact ccagcctggg cgacagagta agactctgtc tcaaaaataa aataaataaa 13740

tcaatcagat attccaatct tttcctttat ttatttattt attttctatt ttggaaacac 13800

agtccttcct tattccagaa ttacacatat attctatttt tctttatatg ctccagtttt 13860

ttttagacct tcacctgaaa tgtgtgtata caaaatctag gccagtccag cagagcctaa 13920

aggtaaaaaa taaaataata aaaaataaat aaaatctagc tcactccttc acatcaaaat 13980

ggagatacag ctgttagcat taaataccaa ataacccatc ttgtcctcaa taattttaag 14040

cgcctctctc caccacatct aactcctgtc aaaggcatgt gccccttccg ggcgctctgc 14100

tgtgctgcca accaactggc atgtggactc tgcagggtcc ctaactgcca agccccacag 14160

tgtgccctga ggctgcccct tccttctagc ggctgccccc actcggcttt gctttcccta 14220

gtttcagtta cttgcgttca gccaaggtct gaaactaggt gcgcacagag cggtaagact 14280

gcgagagaaa gagaccagct ttacaggggg tttatcacag tgcaccctga cagtcgtcag 14340

cctcacaggg ggtttatcac attgcaccct gacagtcgtc agcctcacag ggggtttatc 14400

acagtgcacc cttacaatca ttccatttga ttcacaattt ttttagtctc tactgtgcct 14460

aacttgtaag ttaaatttga tcagaggtgt gttcccagag gggaaaacag tatatacagg 14520

gttcagtact atcgcatttc aggcctccac ctgggtcttg gaatgtgtcc cccgaggggt 14580

gatgactacc tcagttggat ctccacaggt cacagtgaca caagataacc aagacacctc 14640

ccaaggctac cacaatgggc cgccctccac gtgcacatgg ccggaggaac tgccatgtcg 14700

gaggtgcaag cacacctgcg catcagagtc cttggtgtgg agggagggac cagcgcagct 14760

tccagccatc cacctgatga acagaaccta gggaaagccc cagttctact tacaccagga    14820

aaggc                                                                14825

<210>  2
<211>  1003
<212>  PRT
<213>  Human immunodeficiency virus type 1

<400>  2

Phe Phe Arg Glu Asp Leu Ala Phe Pro Gln Gly Lys Ala Arg Glu Phe
1               5                   10                  15

Ser Ser Glu Gln Thr Arg Ala Asn Ser Pro Thr Arg Arg Glu Leu Gln
            20                  25                  30

Val Trp Gly Arg Asp Asn Asn Ser Leu Ser Glu Ala Gly Ala Asp Arg
        35                  40                  45

Gln Gly Thr Val Ser Phe Ser Phe Pro Gln Ile Thr Leu Trp Gln Arg
    50                  55                  60

Pro Leu Val Thr Ile Lys Ile Gly Gly Gln Leu Lys Glu Ala Leu Leu
65                  70                  75                  80

Asp Thr Gly Ala Asp Asp Thr Val Leu Glu Glu Met Asn Leu Pro Gly
                85                  90                  95

Arg Trp Lys Pro Lys Met Ile Gly Gly Ile Gly Gly Phe Ile Lys Val
            100                 105                 110

Arg Gln Tyr Asp Gln Ile Leu Ile Glu Ile Cys Gly His Lys Ala Ile
        115                 120                 125

Gly Thr Val Leu Val Gly Pro Thr Pro Val Asn Ile Ile Gly Arg Asn
    130                 135                 140

Leu Leu Thr Gln Ile Gly Cys Thr Leu Asn Phe Pro Ile Ser Pro Ile
145                 150                 155                 160

Glu Thr Val Pro Val Lys Leu Lys Pro Gly Met Asp Gly Pro Lys Val
                165                 170                 175

Lys Gln Trp Pro Leu Thr Glu Glu Lys Ile Lys Ala Leu Val Glu Ile
            180                 185                 190

Cys Thr Glu Met Glu Lys Glu Gly Lys Ile Ser Lys Ile Gly Pro Glu
        195                 200                 205

Asn Pro Tyr Asn Thr Pro Val Phe Ala Ile Lys Lys Lys Asp Ser Thr
    210                 215                 220

Lys Trp Arg Lys Leu Val Asp Phe Arg Glu Leu Asn Lys Arg Thr Gln
225                 230                 235                 240

Asp Phe Trp Glu Val Gln Leu Gly Ile Pro His Pro Ala Gly Leu Lys
                245                 250                 255

Gln Lys Lys Ser Val Thr Val Leu Asp Val Gly Asp Ala Tyr Phe Ser
            260                 265                 270

Val Pro Leu Asp Lys Asp Phe Arg Lys Tyr Thr Ala Phe Thr Ile Pro
        275                 280                 285

Ser Ile Asn Asn Glu Thr Pro Gly Ile Arg Tyr Gln Tyr Asn Val Leu
    290                 295                 300

Pro Gln Gly Trp Lys Gly Ser Pro Ala Ile Phe Gln Cys Ser Met Thr
305                 310                 315                 320

Lys Ile Leu Glu Pro Phe Arg Lys Gln Asn Pro Asp Ile Val Ile Tyr
                325                 330                 335

Gln Tyr Met Asp Asp Leu Tyr Val Gly Ser Asp Leu Glu Ile Gly Gln
            340                 345                 350

His Arg Thr Lys Ile Glu Glu Leu Arg Gln His Leu Leu Arg Trp Gly
        355                 360                 365

Phe Thr Thr Pro Asp Lys Lys His Gln Lys Glu Pro Pro Phe Leu Trp
    370                 375                 380

Met Gly Tyr Glu Leu His Pro Asp Lys Trp Thr Val Gln Pro Ile Val
385                 390                 395                 400

Leu Pro Glu Lys Asp Ser Trp Thr Val Asn Asp Ile Gln Lys Leu Val
                405                 410                 415

Gly Lys Leu Asn Trp Ala Ser Gln Ile Tyr Ala Gly Ile Lys Val Arg
            420                 425                 430

Gln Leu Cys Lys Leu Leu Arg Gly Thr Lys Ala Leu Thr Glu Val Val
        435                 440                 445

Pro Leu Thr Glu Glu Ala Glu Leu Glu Leu Ala Glu Asn Arg Glu Ile
    450                 455                 460

Leu Lys Glu Pro Val His Gly Val Tyr Tyr Asp Pro Ser Lys Asp Leu
465                 470                 475                 480

Ile Ala Glu Ile Gln Lys Gln Gly Gln Gly Gln Trp Thr Tyr Gln Ile
                485                 490                 495

Tyr Gln Glu Pro Phe Lys Asn Leu Lys Thr Gly Lys Tyr Ala Arg Met
            500                 505                 510

Lys Gly Ala His Thr Asn Asp Val Lys Gln Leu Thr Glu Ala Val Gln
        515                 520                 525

Lys Ile Ala Thr Glu Ser Ile Val Ile Trp Gly Lys Thr Pro Lys Phe
    530                 535                 540

Lys Leu Pro Ile Gln Lys Glu Thr Trp Glu Ala Trp Trp Thr Glu Tyr
545                 550                 555                 560

Trp Gln Ala Thr Trp Ile Pro Glu Trp Glu Phe Val Asn Thr Pro Pro
                565                 570                 575

Leu Val Lys Leu Trp Tyr Gln Leu Glu Lys Glu Pro Ile Ile Gly Ala
            580                 585                 590

Glu Thr Phe Tyr Val Asp Gly Ala Ala Asn Arg Glu Thr Lys Leu Gly
        595                 600                 605

Lys Ala Gly Tyr Val Thr Asp Arg Gly Arg Gln Lys Val Val Pro Leu
    610                 615                 620

Thr Asp Thr Thr Asn Gln Lys Thr Glu Leu Gln Ala Ile His Leu Ala
625                 630                 635                 640

Leu Gln Asp Ser Gly Leu Glu Val Asn Ile Val Thr Asp Ser Gln Tyr
                645                 650                 655

Ala Leu Gly Ile Ile Gln Ala Gln Pro Asp Lys Ser Glu Ser Glu Leu
            660                 665                 670

Val Ser Gln Ile Ile Glu Gln Leu Ile Lys Lys Glu Lys Val Tyr Leu
        675                 680                 685

Ala Trp Val Pro Ala His Lys Gly Ile Gly Gly Asn Glu Gln Val Asp
    690                 695                 700

Lys Leu Val Ser Ala Gly Ile Arg Lys Val Leu Phe Leu Asp Gly Ile
705                 710                 715                 720

Asp Lys Ala Gln Glu Glu His Glu Lys Tyr His Ser Asn Trp Arg Ala
                725                 730                 735

Met Ala Ser Asp Phe Asn Leu Pro Pro Val Val Ala Lys Glu Ile Val
            740                 745                 750

Ala Ser Cys Asp Lys Cys Gln Leu Lys Gly Glu Ala Met His Gly Gln
        755                 760                 765

Val Asp Cys Ser Pro Gly Ile Trp Gln Leu Asp Cys Thr His Leu Glu
    770                 775                 780

Gly Lys Val Ile Leu Val Ala Val His Val Ala Ser Gly Tyr Ile Glu
785                 790                 795                 800

Ala Glu Val Ile Pro Ala Glu Thr Gly Gln Glu Thr Ala Tyr Phe Leu
                805                 810                 815

Leu Lys Leu Ala Gly Arg Trp Pro Val Lys Thr Val His Thr Asp Asn
            820                 825                 830

Gly Ser Asn Phe Thr Ser Thr Thr Val Lys Ala Ala Cys Trp Trp Ala
        835                 840                 845

Gly Ile Lys Gln Glu Phe Gly Ile Pro Tyr Asn Pro Gln Ser Gln Gly
    850                 855                 860

Val Ile Glu Ser Met Asn Lys Glu Leu Lys Lys Ile Ile Gly Gln Val
865                 870                 875                 880

Arg Asp Gln Ala Glu His Leu Lys Thr Ala Val Gln Met Ala Val Phe
                885                 890                 895

Ile His Asn Phe Lys Arg Lys Gly Gly Ile Gly Gly Tyr Ser Ala Gly
            900                 905                 910

Glu Arg Ile Val Asp Ile Ile Ala Thr Asp Ile Gln Thr Lys Glu Leu
        915                 920                 925

Gln Lys Gln Ile Thr Lys Ile Gln Asn Phe Arg Val Tyr Tyr Arg Asp
    930                 935                 940

Ser Arg Asp Pro Val Trp Lys Gly Pro Ala Lys Leu Leu Trp Lys Gly
945                 950                 955                 960

Glu Gly Ala Val Val Ile Gln Asp Asn Ser Asp Ile Lys Val Val Pro
                965                 970                 975

Arg Arg Lys Ala Lys Ile Ile Arg Asp Tyr Gly Lys Gln Met Ala Gly
            980                 985                 990

Asp Asp Cys Val Ala Ser Arg Gln  Asp Glu Asp
        995                 1000

<210>  3
<211>  854
<212>  PRT
<213>  Human immunodeficiency virus type 1

<400>  3

Met Arg Val Lys Glu Lys Tyr Gln His Leu Trp Arg Trp Gly Trp Lys
1               5                   10                  15

Trp Gly Thr Met Leu Leu Gly Ile Leu Met Ile Cys Ser Ala Thr Glu
            20                  25                  30

Lys Leu Trp Val Thr Val Tyr Tyr Gly Val Pro Val Trp Lys Glu Ala
        35                  40                  45

Thr Thr Thr Leu Phe Cys Ala Ser Asp Ala Lys Ala Tyr Asp Thr Glu
    50                  55                  60

Val His Asn Val Trp Ala Thr His Ala Cys Val Pro Thr Asp Pro Asn
65                  70                  75                  80

Pro Gln Glu Val Val Leu Val Asn Val Thr Glu Asn Phe Asn Met Trp
                85                  90                  95

Lys Asn Asp Met Val Glu Gln Met His Glu Asp Ile Ile Ser Leu Trp
            100                 105                 110

Asp Gln Ser Leu Lys Pro Cys Val Lys Leu Thr Pro Leu Cys Val Ser
        115                 120                 125

Leu Lys Cys Thr Asp Leu Lys Asn Asp Thr Asn Thr Asn Ser Ser Ser
    130                 135                 140

Gly Arg Met Ile Met Glu Lys Gly Glu Ile Lys Asn Cys Ser Phe Asn
145                 150                 155                 160

Ile Ser Thr Ser Ile Arg Asp Lys Val Gln Lys Glu Tyr Ala Phe Phe
                165                 170                 175

Tyr Lys Leu Asp Ile Val Pro Ile Asp Asn Thr Ser Tyr Arg Leu Ile
            180                 185                 190

Ser Cys Asn Thr Ser Val Ile Thr Gln Ala Cys Pro Lys Val Ser Phe
        195                 200                 205

Glu Pro Ile Pro Ile His Tyr Cys Ala Pro Ala Gly Phe Ala Ile Leu
    210                 215                 220

Lys Cys Asn Asn Lys Thr Phe Asn Gly Thr Gly Pro Cys Thr Asn Val
225                 230                 235                 240

Ser Thr Val Gln Cys Thr His Gly Ile Arg Pro Val Val Ser Thr Gln
                245                 250                 255

Leu Leu Leu Asn Gly Ser Leu Ala Glu Glu Asp Val Val Ile Arg Ser
            260                 265                 270

Ala Asn Phe Thr Asp Asn Ala Lys Thr Ile Ile Val Gln Leu Asn Thr
        275                 280                 285

Ser Val Glu Ile Asn Cys Thr Arg Pro Asn Asn Asn Thr Arg Lys Ser
    290                 295                 300

Ile Arg Ile Gln Arg Gly Pro Gly Arg Ala Phe Val Thr Ile Gly Lys
305                 310                 315                 320

Ile Gly Asn Met Arg Gln Ala His Cys Asn Ile Ser Arg Ala Lys Trp
                325                 330                 335

Asn Ala Thr Leu Lys Gln Ile Ala Ser Lys Leu Arg Glu Gln Phe Gly
            340                 345                 350

Asn Asn Lys Thr Ile Ile Phe Lys Gln Ser Ser Gly Gly Asp Pro Glu
        355                 360                 365

Ile Val Thr His Ser Phe Asn Cys Gly Gly Glu Phe Phe Tyr Cys Asn
    370                 375                 380

Ser Thr Gln Leu Phe Asn Ser Thr Trp Phe Asn Ser Thr Trp Ser Thr
385                 390                 395                 400

Glu Gly Ser Asn Asn Thr Glu Gly Ser Asp Thr Ile Thr Leu Pro Cys
                405                 410                 415

Arg Ile Lys Gln Phe Ile Asn Met Trp Gln Glu Val Gly Lys Ala Met
            420                 425                 430

Tyr Ala Pro Pro Ile Ser Gly Gln Ile Arg Cys Ser Ser Asn Ile Thr
        435                 440                 445

Gly Leu Leu Leu Thr Arg Asp Gly Gly Asn Asn Asn Asn Gly Ser Glu
    450                 455                 460

Ile Phe Arg Pro Gly Gly Gly Asp Met Arg Asp Asn Trp Arg Ser Glu
465                 470                 475                 480

Leu Tyr Lys Tyr Lys Val Val Lys Ile Glu Pro Leu Gly Val Ala Pro
                485                 490                 495

Thr Lys Ala Lys Arg Arg Val Val Gln Arg Glu Lys Arg Ala Val Gly
            500                 505                 510

Ile Gly Ala Leu Phe Leu Gly Phe Leu Gly Ala Ala Gly Ser Thr Met
        515                 520                 525

Gly Cys Thr Ser Met Thr Leu Thr Val Gln Ala Arg Gln Leu Leu Ser
    530                 535                 540

Asp Ile Val Gln Gln Gln Asn Asn Leu Leu Arg Ala Ile Glu Ala Gln
545                 550                 555                 560

Gln His Leu Leu Gln Leu Thr Val Trp Gly Ile Lys Gln Leu Gln Ala
                565                 570                 575

Arg Ile Leu Ala Val Glu Arg Tyr Leu Lys Asp Gln Gln Leu Leu Gly
            580                 585                 590

Ile Trp Gly Cys Ser Gly Lys Leu Ile Cys Thr Thr Ala Val Pro Trp
        595                 600                 605

Asn Ala Ser Trp Ser Asn Lys Ser Leu Glu Gln Ile Trp Asn Asn Met
    610                 615                 620

Thr Trp Met Glu Trp Asp Arg Glu Ile Asn Asn Tyr Thr Ser Leu Ile
625                 630                 635                 640

His Ser Leu Ile Glu Glu Ser Gln Asn Gln Gln Glu Lys Asn Glu Gln
                645                 650                 655

Glu Leu Leu Glu Leu Asp Lys Trp Ala Ser Leu Trp Asn Trp Phe Asn
            660                 665                 670

Ile Thr Asn Trp Leu Trp Tyr Ile Lys Leu Phe Ile Met Ile Val Gly
        675                 680                 685

Gly Leu Val Gly Leu Arg Ile Val Phe Ala Val Leu Ser Ile Val Asn
    690                 695                 700

Arg Val Arg Gln Gly Tyr Ser Pro Leu Ser Phe Gln Thr His Leu Pro
705                 710                 715                 720

Ile Pro Arg Gly Pro Asp Arg Pro Glu Gly Ile Glu Glu Glu Gly Gly
                725                 730                 735

Glu Arg Asp Arg Asp Arg Ser Ile Arg Leu Val Asn Gly Ser Leu Ala
            740                 745                 750

Leu Ile Trp Asp Asp Leu Arg Ser Leu Cys Leu Phe Ser Tyr His Arg
        755                 760                 765

Leu Arg Asp Leu Leu Leu Ile Val Thr Arg Ile Val Glu Leu Leu Gly
    770                 775                 780

Arg Arg Gly Trp Glu Ala Leu Lys Tyr Trp Trp Asn Leu Leu Gln Tyr
785                 790                 795                 800

Trp Ser Gln Glu Leu Lys Asn Ser Ala Val Asn Leu Leu Asn Ala Thr
                805                 810                 815

Ala Ile Ala Val Ala Glu Gly Thr Asp Arg Val Ile Glu Val Leu Gln
            820                 825                 830

Ala Ala Tyr Arg Ala Ile Arg His Ile Pro Arg Arg Ile Arg Gln Gly
        835                 840                 845

Leu Glu Arg Ile Leu Leu
    850

<210>  4
<211>  500
<212>  PRT
<213>  Human immunodeficiency virus type 1

<400>  4

Met Gly Ala Arg Ala Ser Val Leu Ser Gly Gly Glu Leu Asp Lys Trp
1               5                   10                  15

Glu Lys Ile Arg Leu Arg Pro Gly Gly Lys Lys Gln Tyr Lys Leu Lys
            20                  25                  30

His Ile Val Trp Ala Ser Arg Glu Leu Glu Arg Phe Ala Val Asn Pro
        35                  40                  45

Gly Leu Leu Glu Thr Ser Glu Gly Cys Arg Gln Ile Leu Gly Gln Leu
    50                  55                  60

Gln Pro Ser Leu Gln Thr Gly Ser Glu Glu Leu Arg Ser Leu Tyr Asn
65                  70                  75                  80

Thr Ile Ala Val Leu Tyr Cys Val His Gln Arg Ile Asp Val Lys Asp
                85                  90                  95

Thr Lys Glu Ala Leu Asp Lys Ile Glu Glu Glu Gln Asn Lys Ser Lys
            100                 105                 110

Lys Lys Ala Gln Gln Ala Ala Ala Asp Thr Gly Asn Asn Ser Gln Val
        115                 120                 125

Ser Gln Asn Tyr Pro Ile Val Gln Asn Leu Gln Gly Gln Met Val His
    130                 135                 140

Gln Ala Ile Ser Pro Arg Thr Leu Asn Ala Trp Val Lys Val Val Glu
145                 150                 155                 160

Glu Lys Ala Phe Ser Pro Glu Val Ile Pro Met Phe Ser Ala Leu Ser
                165                 170                 175

Glu Gly Ala Thr Pro Gln Asp Leu Asn Thr Met Leu Asn Thr Val Gly
            180                 185                 190

Gly His Gln Ala Ala Met Gln Met Leu Lys Glu Thr Ile Asn Glu Glu
        195                 200                 205

Ala Ala Glu Trp Asp Arg Leu His Pro Val His Ala Gly Pro Ile Ala
    210                 215                 220

Pro Gly Gln Met Arg Glu Pro Arg Gly Ser Asp Ile Ala Gly Thr Thr
225                 230                 235                 240

Ser Thr Leu Gln Glu Gln Ile Gly Trp Met Thr His Asn Pro Pro Ile
                245                 250                 255

Pro Val Gly Glu Ile Tyr Lys Arg Trp Ile Ile Leu Gly Leu Asn Lys
            260                 265                 270

Ile Val Arg Met Tyr Ser Pro Thr Ser Ile Leu Asp Ile Arg Gln Gly
        275                 280                 285

Pro Lys Glu Pro Phe Arg Asp Tyr Val Asp Arg Phe Tyr Lys Thr Leu
    290                 295                 300

Arg Ala Glu Gln Ala Ser Gln Glu Val Lys Asn Trp Met Thr Glu Thr
305                 310                 315                 320

Leu Leu Val Gln Asn Ala Asn Pro Asp Cys Lys Thr Ile Leu Lys Ala
                325                 330                 335

Leu Gly Pro Gly Ala Thr Leu Glu Glu Met Met Thr Ala Cys Gln Gly
            340                 345                 350

Val Gly Gly Pro Gly His Lys Ala Arg Val Leu Ala Glu Ala Met Ser
        355                 360                 365

Gln Val Thr Asn Pro Ala Thr Ile Met Ile Gln Lys Gly Asn Phe Arg
    370                 375                 380

Asn Gln Arg Lys Thr Val Lys Cys Phe Asn Cys Gly Lys Glu Gly His
385                 390                 395                 400

Ile Ala Lys Asn Cys Arg Ala Pro Arg Lys Lys Gly Cys Trp Lys Cys
                405                 410                 415

Gly Lys Glu Gly His Gln Met Lys Asp Cys Thr Glu Arg Gln Ala Asn
            420                 425                 430

Phe Leu Gly Lys Ile Trp Pro Ser His Lys Gly Arg Pro Gly Asn Phe
        435                 440                 445

Leu Gln Ser Arg Pro Glu Pro Thr Ala Pro Pro Glu Glu Ser Phe Arg
    450                 455                 460

Phe Gly Glu Glu Thr Thr Thr Pro Ser Gln Lys Gln Glu Pro Ile Asp
465                 470                 475                 480

Lys Glu Leu Tyr Pro Leu Ala Ser Leu Arg Ser Leu Phe Gly Ser Asp
                485                 490                 495

Pro Ser Ser Gln
            500

<210>  5
<211>  206
<212>  PRT
<213>  Human immunodeficiency virus type 1

<400>  5

Met Gly Gly Lys Trp Ser Lys Ser Ser Val Ile Gly Trp Pro Ala Val
1               5                   10                  15

Arg Glu Arg Met Arg Arg Ala Glu Pro Ala Ala Asp Gly Val Gly Ala
            20                  25                  30

Val Ser Arg Asp Leu Glu Lys His Gly Ala Ile Thr Ser Ser Asn Thr
        35                  40                  45

Ala Ala Asn Asn Ala Ala Cys Ala Trp Leu Glu Ala Gln Glu Glu Glu
    50                  55                  60

Glu Val Gly Phe Pro Val Thr Pro Gln Val Pro Leu Arg Pro Met Thr
65                  70                  75                  80

Tyr Lys Ala Ala Val Asp Leu Ser His Phe Leu Lys Glu Lys Gly Gly
                85                  90                  95

Leu Glu Gly Leu Ile His Ser Gln Arg Arg Gln Asp Ile Leu Asp Leu
            100                 105                 110

Trp Ile Tyr His Thr Gln Gly Tyr Phe Pro Asp Trp Gln Asn Tyr Thr
        115                 120                 125

Pro Gly Pro Gly Val Arg Tyr Pro Leu Thr Phe Gly Trp Cys Tyr Lys
    130                 135                 140

Leu Val Pro Val Glu Pro Asp Lys Val Glu Glu Ala Asn Lys Gly Glu
145                 150                 155                 160

Asn Thr Ser Leu Leu His Pro Val Ser Leu His Gly Met Asp Asp Pro
                165                 170                 175

Glu Arg Glu Val Leu Glu Trp Arg Phe Asp Ser Arg Leu Ala Phe His
            180                 185                 190

His Val Ala Arg Glu Leu His Pro Glu Tyr Phe Lys Asn Cys
        195                 200                 205

<210>  6

<400>  6
000

<210>  7
<211>  116
<212>  PRT
<213>  Human immunodeficiency virus type 1

<400>  7

<210>  8
<211>  192
<212>  PRT
<213>  Human immunodeficiency virus type 1

<400>  8

Met Glu Asn Arg Trp Gln Val Met Ile Val Trp Gln Val Asp Arg Met
1               5                   10                  15

Arg Ile Asn Thr Trp Lys Arg Leu Val Lys His His Met Tyr Ile Ser
            20                  25                  30

Arg Lys Ala Lys Asp Trp Phe Tyr Arg His His Tyr Glu Ser Thr Asn
        35                  40                  45

Pro Lys Ile Ser Ser Glu Val His Ile Pro Leu Gly Asp Ala Lys Leu
    50                  55                  60

Val Ile Thr Thr Tyr Trp Gly Leu His Thr Gly Glu Arg Asp Trp His
65                  70                  75                  80

Leu Gly Gln Gly Val Ser Ile Glu Trp Arg Lys Lys Arg Tyr Ser Thr
                85                  90                  95

Gln Val Asp Pro Asp Leu Ala Asp Gln Leu Ile His Leu His Tyr Phe
            100                 105                 110

Asp Cys Phe Ser Glu Ser Ala Ile Arg Asn Thr Ile Leu Gly Arg Ile
        115                 120                 125

Val Ser Pro Arg Cys Glu Tyr Gln Ala Gly His Asn Lys Val Gly Ser
    130                 135                 140

Leu Gln Tyr Leu Ala Leu Ala Ala Leu Ile Lys Pro Lys Gln Ile Lys
145                 150                 155                 160

Pro Pro Leu Pro Ser Val Arg Lys Leu Thr Glu Asp Arg Trp Asn Lys
                165                 170                 175

Pro Gln Lys Thr Lys Gly His Arg Gly Ser His Thr Met Asn Gly His
            180                 185                 190

<210> 9
<211> 81
<212> PRT
<213> Human immunodeficiency virus type 1

<400> 9

Met Gln Pro Ile Ile Val Ala Ile Val Ala Leu Val Val Ala Ile Ile
1               5                   10                  15

Ile Ala Ile Val Val Trp Ser Ile Val Ile Ile Glu Tyr Arg Lys Ile
            20                  25                  30

Leu Arg Gln Arg Lys Ile Asp Arg Leu Ile Asp Arg Leu Ile Glu Arg
        35                  40                  45

Ala Glu Asp Ser Gly Asn Glu Ser Glu Gly Glu Val Ser Ala Leu Val
    50                  55                  60

Glu Met Gly Val Glu Met Gly His His Ala Pro Trp Asp Ile Asp Asp
65                  70                  75                  80

Leu

<210>  10
<211>  96
<212>  PRT
<213>  Human immunodeficiency virus type 1

<400>  10

Met Glu Gln Ala Pro Glu Asp Gln Gly Pro Gln Arg Glu Pro Tyr Asn
1               5                   10                  15

Glu Trp Thr Leu Glu Leu Leu Glu Glu Leu Lys Ser Glu Ala Val Arg
            20                  25                  30

His Phe Pro Arg Ile Trp Leu His Asn Leu Gly Gln His Ile Tyr Glu
        35                  40                  45

Thr Tyr Gly Asp Thr Trp Ala Gly Val Glu Ala Ile Ile Arg Ile Leu
    50                  55                  60

Gln Gln Leu Leu Phe Ile His Phe Arg Ile Gly Cys Arg His Ser Arg
65                  70                  75                  80

Ile Gly Val Thr Arg Gln Arg Arg Ala Arg Asn Gly Ala Ser Arg Ser
                85                  90                  95

<210>  11
<211>  33
<212>  DNA
<213>  Artificial Sequence

<220>
<223>  cebador 52EU

<400>  11
gctataagat gggtggcgcg gccgcaaaaa gta                                    33


<210>  12
<211>  33
<212>  DNA
<213>  Artificial Sequence

<220>
<223>  cebador 104ED

<400>  12
agctggatcc gtctcgagat actgctccca ccc                                    33


<210>  13
<211>  311
<212>  PRT
<213>  Renilla reniformis

<400>  13

Met Thr Ser Lys Val Tyr Asp Pro Glu Gln Arg Lys Arg Met Ile Thr
1               5                   10                  15


Gly Pro Gln Trp Trp Ala Arg Cys Lys Gln Met Asn Val Leu Asp Ser
            20                  25                  30


Phe Ile Asn Tyr Tyr Asp Ser Glu Lys His Ala Glu Asn Ala Val Ile
        35                  40                  45


Phe Leu His Gly Asn Ala Ala Ser Ser Tyr Leu Trp Arg His Val Val
    50                  55                  60


Pro His Ile Glu Pro Val Ala Arg Cys Ile Ile Pro Asp Leu Ile Gly
65                  70                  75                  80


Met Gly Lys Ser Gly Lys Ser Gly Asn Gly Ser Tyr Arg Leu Leu Asp
                85                  90                  95


His Tyr Lys Tyr Leu Thr Ala Trp Phe Glu Leu Leu Asn Leu Pro Lys
            100                 105                 110


Lys Ile Ile Phe Val Gly His Asp Trp Gly Ala Cys Leu Ala Phe His
        115                 120                 125


Tyr Ser Tyr Glu His Gln Asp Lys Ile Lys Ala Ile Val His Ala Glu
    130                 135                 140

Ser Val Val Asp Val Ile Glu Ser Trp Asp Glu Trp Pro Asp Ile Glu
145                 150                 155                 160

Glu Asp Ile Ala Leu Ile Lys Ser Glu Glu Gly Glu Lys Met Val Leu
                165                 170                 175

Glu Asn Asn Phe Phe Val Glu Thr Met Leu Pro Ser Lys Ile Met Arg
            180                 185                 190

Lys Leu Glu Pro Glu Glu Phe Ala Ala Tyr Leu Glu Pro Phe Lys Glu
        195                 200                 205

Lys Gly Glu Val Arg Arg Pro Thr Leu Ser Trp Pro Arg Glu Ile Pro
    210                 215                 220

Leu Val Lys Gly Gly Lys Pro Asp Val Val Gln Ile Val Arg Asn Tyr
225                 230                 235                 240

Asn Ala Tyr Leu Arg Ala Ser Asp Asp Leu Pro Lys Met Phe Ile Glu
                245                 250                 255

Ser Asp Pro Gly Phe Phe Ser Asn Ala Ile Val Glu Gly Ala Lys Lys
            260                 265                 270

Phe Pro Asn Thr Glu Phe Val Lys Val Lys Gly Leu His Phe Ser Gln
        275                 280                 285

Glu Asp Ala Pro Asp Glu Met Gly Lys Tyr Ile Lys Ser Phe Val Glu
    290                 295                 300

Arg Val Leu Lys Asn Glu Gln
305                 310

<210>  14
<211>  1024
<212>  PRT
<213>  Escherichia coli

<400>  14

Met Thr Met Ile Thr Asp Ser Leu Ala Val Val Leu Gln Arg Arg Asp
1               5                   10                  15

Trp Glu Asn Pro Gly Val Thr Gln Leu Asn Arg Leu Ala Ala His Pro
            20                  25                  30

Pro Phe Ala Ser Trp Arg Asn Ser Glu Glu Ala Arg Thr Asp Arg Pro
        35                  40                  45

Ser Gln Gln Leu Arg Ser Leu Asn Gly Glu Trp Arg Phe Ala Trp Phe
    50                  55                  60

Pro Ala Pro Glu Ala Val Pro Glu Ser Trp Leu Glu Cys Asp Leu Pro
65                  70                  75                  80

Glu Ala Asp Thr Val Val Val Pro Ser Asn Trp Gln Met His Gly Tyr
                85                  90                  95

Asp Ala Pro Ile Tyr Thr Asn Val Thr Tyr Pro Ile Thr Val Asn Pro
            100                 105                 110

Pro Phe Val Pro Thr Glu Asn Pro Thr Gly Cys Tyr Ser Leu Thr Phe
        115                 120                 125

Asn Val Asp Glu Ser Trp Leu Gln Glu Gly Gln Thr Arg Ile Ile Phe
    130                 135                 140

Asp Gly Val Asn Ser Ala Phe His Leu Trp Cys Asn Gly Arg Trp Val
145                 150                 155                 160

Gly Tyr Gly Gln Asp Ser Arg Leu Pro Ser Glu Phe Asp Leu Ser Ala
                165                 170                 175

Phe Leu Arg Ala Gly Glu Asn Arg Leu Ala Val Met Val Leu Arg Trp
            180                 185                 190

Ser Asp Gly Ser Tyr Leu Glu Asp Gln Asp Met Trp Arg Met Ser Gly
        195                 200                 205

Ile Phe Arg Asp Val Ser Leu Leu His Lys Pro Thr Thr Gln Ile Ser
    210                 215                 220

Asp Phe His Val Ala Thr Arg Phe Asn Asp Asp Phe Ser Arg Ala Val
225                 230                 235                 240

Leu Glu Ala Glu Val Gln Met Cys Gly Glu Leu Arg Asp Tyr Leu Arg
                245                 250                 255

Val Thr Val Ser Leu Trp Gln Gly Glu Thr Gln Val Ala Ser Gly Thr
            260                 265                 270

Ala Pro Phe Gly Gly Glu Ile Ile Asp Glu Arg Gly Gly Tyr Ala Asp
        275                 280                 285

Arg Val Thr Leu Arg Leu Asn Val Glu Asn Pro Lys Leu Trp Ser Ala
    290                 295                 300

Glu Ile Pro Asn Leu Tyr Arg Ala Val Val Glu Leu His Thr Ala Asp
305                 310                 315                 320

Gly Thr Leu Ile Glu Ala Glu Ala Cys Asp Val Gly Phe Arg Glu Val
                325                 330                 335

Arg Ile Glu Asn Gly Leu Leu Leu Leu Asn Gly Lys Pro Leu Leu Ile
            340                 345                 350

Arg Gly Val Asn Arg His Glu His His Pro Leu His Gly Gln Val Met
        355                 360                 365

Asp Glu Gln Thr Met Val Gln Asp Ile Leu Leu Met Lys Gln Asn Asn
    370                 375                 380

Phe Asn Ala Val Arg Cys Ser His Tyr Pro Asn His Pro Leu Trp Tyr
385                 390                 395                 400

Thr Leu Cys Asp Arg Tyr Gly Leu Tyr Val Val Asp Glu Ala Asn Ile
                405                 410                 415

Glu Thr His Gly Met Val Pro Met Asn Arg Leu Thr Asp Asp Pro Arg
            420                 425                 430

Trp Leu Pro Ala Met Ser Glu Arg Val Thr Arg Met Val Gln Arg Asp
        435                 440                 445

Arg Asn His Pro Ser Val Ile Ile Trp Ser Leu Gly Asn Glu Ser Gly
    450                 455                 460

His Gly Ala Asn His Asp Ala Leu Tyr Arg Trp Ile Lys Ser Val Asp
465                 470                 475                 480

Pro Ser Arg Pro Val Gln Tyr Glu Gly Gly Gly Ala Asp Thr Thr Ala
                485                 490                 495

Thr Asp Ile Ile Cys Pro Met Tyr Ala Arg Val Asp Glu Asp Gln Pro
            500                 505                 510

Phe Pro Ala Val Pro Lys Trp Ser Ile Lys Lys Trp Leu Ser Leu Pro
        515                 520                 525

Gly Glu Thr Arg Pro Leu Ile Leu Cys Glu Tyr Ala His Ala Met Gly
    530                 535                 540

Asn Ser Leu Gly Gly Phe Ala Lys Tyr Trp Gln Ala Phe Arg Gln Tyr
545                 550                 555                 560

Pro Arg Leu Gln Gly Gly Phe Val Trp Asp Trp Val Asp Gln Ser Leu
                565                 570                 575

Ile Lys Tyr Asp Glu Asn Gly Asn Pro Trp Ser Ala Tyr Gly Gly Asp
            580                 585                 590

Phe Gly Asp Thr Pro Asn Asp Arg Gln Phe Cys Met Asn Gly Leu Val
        595                 600                 605

Phe Ala Asp Arg Thr Pro His Pro Ala Leu Thr Glu Ala Lys His Gln
    610                 615                 620

Gln Gln Phe Phe Gln Phe Arg Leu Ser Gly Gln Thr Ile Glu Val Thr
625                 630                 635                 640

Ser Glu Tyr Leu Phe Arg His Ser Asp Asn Glu Leu Leu His Trp Met
                645                 650                 655

Val Ala Leu Asp Gly Lys Pro Leu Ala Ser Gly Glu Val Pro Leu Asp
            660                 665                 670

Val Ala Pro Gln Gly Lys Gln Leu Ile Glu Leu Pro Glu Leu Pro Gln
        675                 680                 685

Pro Glu Ser Ala Gly Gln Leu Trp Leu Thr Val Arg Val Val Gln Pro
    690                 695                 700

Asn Ala Thr Ala Trp Ser Glu Ala Gly His Ile Ser Ala Trp Gln Gln
705                 710                 715                 720

Trp Arg Leu Ala Glu Asn Leu Ser Val Thr Leu Pro Ala Ala Ser His
                725                 730                 735

Ala Ile Pro His Leu Thr Thr Ser Glu Met Asp Phe Cys Ile Glu Leu
            740                 745                 750

Gly Asn Lys Arg Trp Gln Phe Asn Arg Gln Ser Gly Phe Leu Ser Gln
        755                 760                 765

Met Trp Ile Gly Asp Lys Lys Gln Leu Leu Thr Pro Leu Arg Asp Gln
    770                 775                 780

Phe Thr Arg Ala Pro Leu Asp Asn Asp Ile Gly Val Ser Glu Ala Thr
785                 790                 795                 800

Arg Ile Asp Pro Asn Ala Trp Val Glu Arg Trp Lys Ala Ala Gly His
                805                 810                 815

Tyr Gln Ala Glu Ala Ala Leu Leu Gln Cys Thr Ala Asp Thr Leu Ala
            820                 825                 830

Asp Ala Val Leu Ile Thr Thr Ala His Ala Trp Gln His Gln Gly Lys
        835                 840                 845

Thr Leu Phe Ile Ser Arg Lys Thr Tyr Arg Ile Asp Gly Ser Gly Gln
    850                 855                 860

Met Ala Ile Thr Val Asp Val Glu Val Ala Ser Asp Thr Pro His Pro
865                 870                 875                 880

Ala Arg Ile Gly Leu Asn Cys Gln Leu Ala Gln Val Ala Glu Arg Val
                885                 890                 895

Asn Trp Leu Gly Leu Gly Pro Gln Glu Asn Tyr Pro Asp Arg Leu Thr
            900                 905                 910

Ala Ala Cys Phe Asp Arg Trp Asp Leu Pro Leu Ser Asp Met Tyr Thr
        915                 920                 925

Pro Tyr Val Phe Pro Ser Glu Asn Gly Leu Arg Cys Gly Thr Arg Glu
    930                 935                 940

Leu Asn Tyr Gly Pro His Gln Trp Arg Gly Asp Phe Gln Phe Asn Ile
945                 950                 955                 960

Ser Arg Tyr Ser Gln Gln Gln Leu Met Glu Thr Ser His Arg His Leu
                965                 970                 975

Leu His Ala Glu Glu Gly Thr Trp Leu Asn Ile Asp Gly Phe His Met
            980                 985                 990

Gly Ile Gly Gly Asp Asp Ser Trp  Ser Pro Ser Val Ser  Ala Glu Phe
        995                 1000                1005

Gln Leu  Ser Ala Gly Arg Tyr  His Tyr Gln Leu Val  Trp Cys Gln
    1010                1015                1020

Lys

<210>  15
<211>  34
<212>  DNA
<213>  Artificial Sequence

<220>
<223>  cebador NarI-LacZ-UP

<400>  15
gcagtggcgc cgcgcccaat acgcaaaccg cctc                                34

<210>  16
<211>  35
<212>  DNA
<213>  Artificial Sequence

<220>
<223>  cebador ApaI-lacZ-D

<400>  16
tcctaggggc ccgccattcg ccattcaggc tgcgc                               35

<210>  17

<211>  34
<212>  DNA
<213>  Artificial Sequence

<220>
<223>  cebador M-KspI-UP

<400>  17
tacaagggac tttccgcggg ggactttcca ggga                                    34


<210>  18
<211>  34
<212>  DNA
<213>  Artificial Sequence

<220>
<223>  cebador M-KspI-Down

<400>  18
tccctggaaa gtcccccgcg gaaagtccct tgta                                    34


<210>  19
<211>  36
<212>  DNA
<213>  Artificial Sequence

<220>
<223>  cebador NotI-LacZ-UP

<400>  19
gtggcgcggc cgcgcgccca atacgcaaac cgcctc                                  36


<210>  20
<211>  35
<212>  DNA
<213>  Artificial Sequence

<220>
<223>  cebador KspI-LacZ-D

<400>  20
agtcccccgc gggccattcg ccattcaggc tgcgc                                   35


<210>  21
<211>  45
<212>  DNA
<213>  Artificial Sequence

<220>
<223>  cebador Mut-NcoI-Env-UP

<400>  21
gtagtagcaa taataatagc catggttgtg tggtccatag taatc                        45

<210>  22
<211>  45
<212>  DNA
<213>  Artificial Sequence

<220>
<223>  cebador Mut-NcoI-Env-down

<400>  22
gattactatg gaccacacaa ccatggctat tattattgct actac                        45


<210>  23
<211>  34
<212>  DNA
<213>  Artificial Sequence

<220>
<223>  cebador 127

<400>  23
gcagtccatg ggcgcccaat acgcaaaccg cctc                                    34


<210>  24
<211>  35
<212>  DNA
<213>  Artificial Sequence

<220>
<223>  cebador Not-LacZ-down

<400>  24
ttttgcggcc gcgccattgc ccattcaggc tgcgc                                   35


<210>  25
<211>  38
<212>  DNA
<213>  Artificial Sequence

<220>
<223>  cebador Mut-AgeI-UP

<400>  25
gtcctattga gactgtaccg gtaaaattaa agccagcc                                38


<210>  26
<211>  38
<212>  DNA
<213>  Artificial Sequence

<220>
<223>  cebador Mut-AgeI-Down

<400>  26
ggctggcttt aattttaccg gtacagtctc aataggac                              38


<210>  27
<211>  36
<212>  DNA
<213>  Artificial Sequence

<220>
<223>  cebador AgeI-lacZ-D

<400>  27
tcctagaccg gtgccattcg ccattcaggc tgctgc                                36


<210>  28
<211>  24
<212>  DNA
<213>  Artificial Sequence

<220>
<223>  cebador 107 localizado en pNL4-3 entre los nucleótidos 560 y 584

<400>  28
gtgcccgtct gttgtgtgac tctg                                             24


<210>  29
<211>  24
<212>  DNA
<213>  Artificial Sequence

<220>
<223>  cebador 122 localizado en pNL4-3 entre los nucleótidos 2080 y
       2106

<400>  29
cctgtctctc agtacaatct ttca                                             24


<210>  30
<211>  29
<212>  DNA
<213>  Artificial Sequence

<220>
<223>  cebador 163 localizado en pNL4-3 entre los nucleótidos 621 y 648

<400>  30
gaaaatctct agcagtggcg cccgaacag                                        29


<210>  31
<211>  24
<212>  DNA

<213>  Artificial Sequence

<220>
<223>  cebador 123 localizado en pNL4-3 entre los nucleótidos 2017 y
      2042

<400>  31
tcctaggggc cctgcaattt ttgg                                              24


<210>  32
<211>  24
<212>  DNA
<213>  Artificial Sequence

<220>
<223>  cebador 119 localizado en pNL4-3 entre los nucleótidos 8739 y
      8763

<400>  32
acatacctag aagaataaga cagg                                              24


<210>  33
<211>  24
<212>  DNA
<213>  Artificial Sequence

<220>
<223>  cebador 121localizado en pNL4-3 entre los nucleótidos 9443 y 9467

<400>  33
gtcccccgcg gaaagtccct tgta                                              24


<210>  34
<211>  26
<212>  DNA
<213>  Artificial Sequence

<220>
<223>  cebador 118 localizado en pNL4-3 entre los nucleótidos 9622 y
      9648

<400>  34
gcactcaagg caagctttat tgaggc                                            26


<210>  35
<211>  26
<212>  DNA
<213>  Artificial Sequence

<220>
<223>  cebador 117 localizado en pNL4-3 entre los nucleótidos 8678 y
      8704

<400>  35
gtagctgagg ggacagatag ggttat                                          26


<210>  36
<211>  24
<212>  DNA
<213>  Artificial Sequence

<220>
<223>  cebador 102 localizado en pNL4-3 entre los nucleótidos 8927 y
       8950

<400>  36
ttgctacttg tgattgctcc atgt                                            24


<210>  37
<211>  25
<212>  DNA
<213>  Artificial Sequence

<220>
<223>  cebador 101 localizado en pNL4-3 entre los nucleótidos 5852 y
       5876

<400>  37
tagagccctg gaagcatcca ggaag                                           25


<210>  38
<211>  34
<212>  DNA
<213>  Artificial Sequence

<220>
<223>  cebador 161 localizado a en pNL4-3 entre los nucleótidos 8780 y
       8812

<400>  38
tactttttgc ggccgcgcca cccaatctta tagc                                 34


<210>  39
<211>  39
<212>  DNA
<213>  Artificial Sequence

<220>
<223>  cebador 126

<400>  39
gccatggawg tgtggaccat agtawwcata gaatatarg                            39


<210>  40
<211>  20

<212>  DNA
<213>  Artificial Sequence

<220>
<223>  cebador 125 localizado en pNL4-3 entre los nucleótidos 3623 y
       3643

<400>  40
acatcattag tgtgggcacc                                                    20


<210>  41
<211>  34
<212>  DNA
<213>  Artificial Sequence

<220>
<223>  cebador 159 localizado en pNL4-3 entre los nucleótidos 3467 y
       3500

<400>  41
cactccatgt accggttctt ttagaatctc cctg                                    34

## Description

### Technical Field of the Invention

The invention is encompassed in the field of treating viral diseases, more specifically in preventing and/or treating infection caused by the human immunodeficiency virus (HIV) by means of using non-replicative virions.

### Background of the Invention

UNAIDS (Joint United Nation Programme on HIV/AIDS) estimates that human immunodeficiency virus (HIV) infection has caused more than 20 million deaths since its appearance in 1981 and the worldwide figure of infected persons at the end of 2008 was about 40 million, mainly (95%) in developing countries. It is one of the infectious diseases which causes the most deaths worldwide and it is calculated that 14,000 new infections (5 million/year) take place everyday. In the United States and Western Europe, the morbidity and mortality due to HIV infection have reduced spectacularly after the introduction of Highly Active Antiretroviral Therapy (HAART).

Besides its high efficiency for controlling viral replication and for restoring immunity against opportunistic infections, HAART is incapable of eradicating the virus and recovering the specific responses against HIV in HIV-infected persons. In fact, after withdrawing HAART a rapid increase of viral load occurs in all cases (Carcelain et al., 2001. J Virol 75(1):234-41). This implies that once HAART is started it must be continued throughout life, which is problematic due to the different types of serious side effects which it may involve, such as metabolic alterations and the cardiovascular risk that it entails.

Currently, the therapeutic arsenal against HIV infection is very complete and there are perspectives that it will continue to increase in a significant manner in the upcoming years. This situation is in contrast with the limited advances obtained in the development of a preventive or therapeutic vaccine. This is due to, among other reasons, the genetic diversity, the fast replication rate and the high mutation frequency inherent to HIV (Autran et al., 2003. Nat Rev Immunol 3(6):503-8), the difficulty for inducing neutralizing antibodies, the lack of a suitable animal model, the limited knowledge of the immune response capable of generating protection against HIV infection and the different transmission routes of the virus (Kaufmann et al., 2005. Nat Med. 11(4 Suppl):S33-44).

A future objective for vaccine design consists of increasing their efficiency by enabling their accessibility to the greatest number of antigen presenting cells (APCs) possible and achieving high local concentrations required to induce strong immunological responses. By facilitating the proper penetration of vaccine compounds into the APC-rich immunization sites as well as the specific migration and activation of APCs, the efficiency of the new vaccines in inducting protective immune responses would be increased. The vaccines could be efficient for inducing strong and protective immune responses against pathogens if they penetrate properly and activate the dendritic cells (DCs) which play a unique role as inducers of antigen specific responses. In the case of HIV infection, the clearance of the virus depends on the generation of antibodies but also on the induction of effector and memory T cells, CD4+ and CD8+. Different in depth studies on memory immune responses have been focused on the importance of forming a functional pool of memory CD8+ T cells for controlling the viral infection and requiring a suitable function of the APCs and of CD4+ T cell responses.

Currently, there is no preventive or therapeutic vaccine approved for use in HIV. None of the therapeutic vaccines used have shown long term efficacy with respect to the control of viral replication, the increase of CD4 lymphocytes or the progression to AIDS in randomized studies. Vaccines from attenuated live viruses have traditionally been used to prevent viral infections, such as, for example, measles, mumps or rubella. These vaccines are highly immunogenic because they imitate the natural infection both in the cell and humoral response (McMichael and Hanke, 2003. Nat Med. 9:874-80), and they induce life-long immunity after one or two doses. In contrast, vaccines using dead organisms or subunits do not imitate the infection and the administration of these vaccines only induces a humoral response, periodic boosters are required to maintain the antibodies at suitable concentrations, and furthermore adjuvants such as aluminium salts are used to increase these responses which are at times very poor.

Some of these traditional vaccine strategies cannot be used to prevent HIV infection or therapeutically due to safety problems (Mwau, McMichael. 2003. J Gene Med. 5:3-10) or because they have had nil efficacy when used (Moss et al., 2000. Vaccine. 18:1081-87). For example, attenuated live vaccines are prohibited in humans since in experiments with animals the highly attenuated simian immunodeficiency virus (SIV) protecting adult macaques from HIV infection caused AIDS in infant macaques, furthermore some adult macaques subsequently developed AIDS (Wyand et al., 1997. Nat Med.; 3:32-36). Moreover, the degree of attenuation was inversely correlated with immunogenicity (Wyand et al. 1997. Nat. Med.; 3:32-36). In addition, the vaccines of dead viruses have given null results in clinical trials (Moss et al. 2000. Vaccine. 18:1081-87).

Therefore, the development of new effective and safe HIV vaccines is necessary.

### Summary of the Invention

The authors of the present invention have developed viral vectors comprising an HIV virus genome as well as non-replicative virions capable of generating an immune response.

In a first aspect, the invention relates to a viral vector comprising a human immunodeficiency virus (HIV) genome °containing a mutation in the *pol* gene in which said mutation causes the virus produced solely from said viral vector to be a non-replicative virus.

In another additional aspect, the invention relates to a non-replicative virion comprising a viral vector of the invention.

In another additional aspect, the invention relates to a method for obtaining an activated immune cell which comprises the steps of:
a) contacting an immune cell with a non-replicative virion of the invention,
b) maintaining the mixture obtained in step a) in conditions suitable for infecting said immune cells with the virion and for processing the antigens originating from the non-replicative virion, and
c) optionally, recovering the activated immune cells.

In another additional aspect, the invention relates to an *in vitro* method for obtaining a customized HIV vaccine for an HIV-infected subject, wherein said vaccine comprises activated immune cells, which comprises:
a) contacting immune cells *in vitro* with a non-replicative virion of the invention comprising a viral vector of the invention in which the genome of said second HIV originates from an HIV of said HIV-infected subject;
b) maintaining the mixture obtained in step a) in conditions suitable for infecting said cells with the virion and for processing the antigens originating from the non-replicative virion;
c) detecting and quantifying the activation of the immune cells activated according to steps a) and b);
d) comparing the activation of said immune cells activated according to steps a) and b) with the activation of control immune cells,
e) selecting the virions which cause a greater activation of the immune cells activated according to steps a) and b) than the activation of the control immune cells,
f) infecting immune cells of said HIV-infected subject with the non-replicative virions selected in step e), and
g) recovering the immune cells activated according to step f).

In another additional aspect, the invention relates to an *in vitro* method for obtaining a customized HIV vaccine for an HIV-infected subject, wherein said vaccine comprises activated immune cells, which comprises:
a) contacting immune cells originating from said HIV-infected subject *in vitro* with a non-replicative virion comprising a viral vector of the invention in which the genome of said second HIV originates from an HIV of said HIV-infected subject;
b) maintaining the mixture obtained in step a) in conditions suitable for infecting said immune cells with the virion and for processing the antigens originating from the non-replicative virion; and
c) recovering the immune cells activated according to step b).

In another aspect, the invention relates to a cell obtainable according to any of the methods of the invention.

In another additional aspect, the invention relates to the use of the non-replicative virion of the invention or of the cell of the invention in medicine.

In another additional aspect, the invention relates to the use of the non-replicative virion of the invention or of the cell of the invention in the preparation of an HIV vaccine.

In another additional aspect, the invention relates to the use of the non-replicative virion of the invention or of the cell of the invention in the preparation of a pharmaceutical composition for treating AIDS

### Brief Description of the Drawings

Figure 1 A shows the schematic depiction of the HIV genome pNL4-3. B. Schematic depiction of pNL4-3 ART. C. Schematic depiction of viral vectors pNL4-3 ΔRT/Δgag- [*LacZ*], pNL4-3 ΔRT/Δ*env*-[*LacZ*], pNL4-3 ΔRT/Δ*env-nef*-[*LacZ*], pNL4-3 ΔRT/Δ*nef-[LacZ]* and pNL4-3 ΔRT/Δ*gag-pro*-[*LacZ*], wherein the *LacZ* (beta-galactosidase) gene is substituting the gene fragments corresponding to the *gag, env, env-nef, nef* or *gag-pro* genes, respectively

Figure 2 shows the measurement of luciferase activity to analyze the infection/replication capacity of the NL4-3 Ren WT, heat-inactivated NL4-3 Ren WT virions or NL4-3 ΔRT Ren (NL4-3 DRT Ren) virions after 24 or 96 hours post-infection of MT-2 cells. *Ren:* renilla gene, WT: wild-type strain.

Figure 3 shows the measurement of luciferase activity to analyze the replicative capacity of the NL4-3 ΔRT (deltaRT) and NL4-3 (WT) virions after 72 hours post-infection of TZM-bl cells.

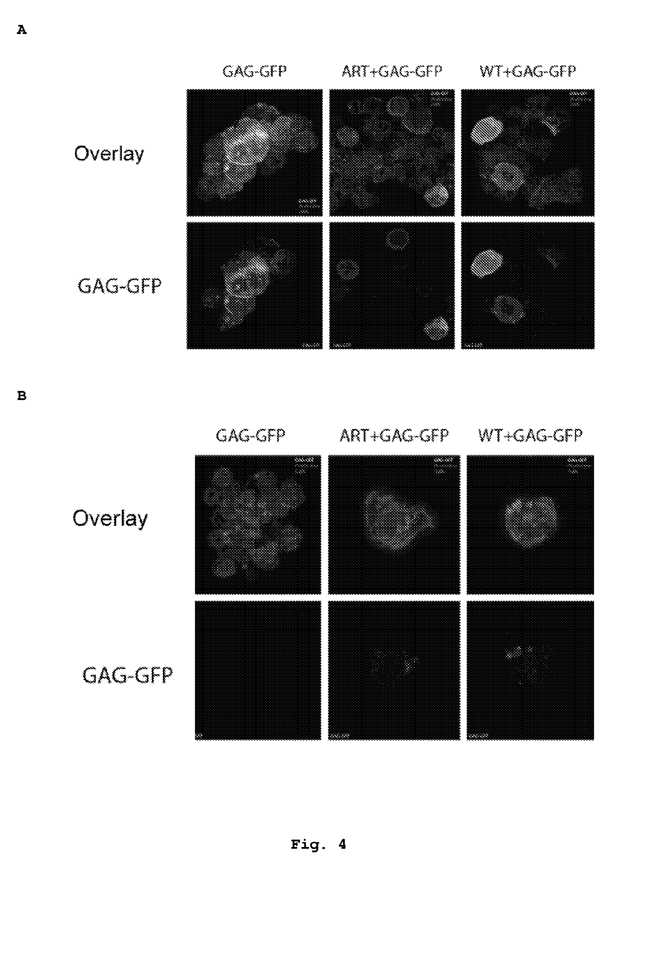

Figure 4 A shows 293T cells co-transfected with pGAG-GFP (5 µg), pNL4-3 ΔRT (3 µg) + pGAG-GFP (2 µg) and pNL4-3WT (3 µg) + pGAG-GFP (2 µg) . Upper panel, image overlay showing the cell nucleus, the cortical actin of the cell and the signal visualization of the expression of GAG-GFP. Lower panel, visualization of the expression of GAG-GFP by means of confocal microscopy. B. Infection of PBMC cells (peripheral mononuclear cells) with the virions obtained after co-transfecting 293T with pGAG-GFP (5 µg), pNL4-3WT (3 µg) + pGAG-GFP (2 µg) and pNL4-3 ΔRT (3 µg) + pGAG-GFP (2 µg). Upper panel, image overlay showing the cell nucleus, the cortical actin of the cell and the expression of GAG-GFP. Lower panel, visualization of the location of the GAG-GFP signal by means of confocal microscopy.

Figure 5 shows the visualization by means of transmission electron microscopy of the NL4-3 (A) and NL4-3 ΔRT (B) virion-producing 293T cells. Virions purified from NL4-3 (C) and NL4-3 ΔRT (D).

Figure 6 shows the Western blot of protein extracts NL4-3 and NL4-3 ΔRT virions and of the 293T cells transfected with pNL4-3 (NL4-3) and pNL4-3 ΔRT (ART).

Figure 7 shows the sequential scheme of the *ex vivo* model of the MD-DC assay.

Figure 8 shows the assessment of the cellular response of the MD-DC cells against different immunogens: BaL30 (heat-inactivated R5 strain of HIV-1), heat-inactivated recombinant p24, NL4-3 WT or NL4-3 ΔRT in an HIV patient. The proliferation index (S.I.) (A), and the production of IL-2 (B) and interferon γ (IFN _{Y}) (C) are quantified.

Figure 9 shows the results of the cellular response obtained in the ex *vivo* model of MD-DC pulsed with the following immunogens: NL4-3 WT (heat-inactivated) or NL4-3 ART. Test performed with 8 responding patients and against one and the same stock of immunogens with a degree of significance 0.01 **.

Figure 10 shows the comparison of the results obtained using the MD-DC co-culture model and the *ex vivo* model established with PBMC.

Figure 11 shows the visualization by means of electron microscopy of purified NL4-3 virions obtained by transfecting 293T cells in the absence (A) or in the presence (B) of Amprenavir (protease inhibitor).

### Detailed Description of the Invention

The authors of the present invention have developed viral vectors comprising a human immunodeficiency virus (HIV) genome, as well as non-replicative virions comprising said viral vectors for the purpose of obtaining autologous and heterologous vaccines, both preventive and therapeutic, against HIV/AIDS infection. Thus, as shown in Example 3, the virions of the invention are capable of generating an immune response.

In a first aspect, the invention relates to a viral vector, hereinafter viral vector of the invention, comprising a human immunodeficiency virus (HIV) genome containing a mutation in the *pol* gene, wherein said mutation causes the virus produced solely from said viral vector to be a non-replicative virus.

As it is used herein, "viral vector" refers to a nucleic acid sequence comprising the necessary sequences so that after transcribing and translating said sequences in a cell a viral particle with capacity for infecting a new cell is generated. The vectors of the invention can be obtained by means of techniques widely known in the state of the art [Sambrook et al., "Molecular cloning, a Laboratory Manual", 2nd ed., Cold Spring Harbor Laboratory Press, N.Y., 1989 Vol 1-3].

As it is used herein, "HIV" is intended to include HIV-1 and HIV-2. "HIV-1" means the type 1 human immunodeficiency virus. HIV-1 includes but is not limited to extracellular virus particles and the forms of HIV-1 associated with HIV-1-infected cells. "HIV-2" means the type 2 human immunodeficiency virus. HIV-2 includes but is not limited to extracellular virus particles and the forms of HIV-2 associated with HIV-2-infected cells.

In a particular embodiment, the WT (wild-type) HIV genome that has been used for obtaining the viral vectors of the invention corresponds to the HIV-1 genome pNL4-3, NIH AIDS Research & Reference Reagent Program, no. 114, GenBank database accession number M19921.1 (SEQ ID NO: 1) as of 1st December 2010.

As it is used herein, the term "mutation" refers to a change in a nucleic acid sequence. Said mutation can include, among others, substitution, i.e., exchanging one or more nucleotides for others; inversion, i.e., a DNA segment inside a gene is inverted, to that end two 180° rotations are necessary, one for inverting the sequence and the other for maintaining the polarity of the DNA; translocation, i.e., a segment of a gene changes position to be in another different site of the same gene or in another site of the genome; nucleotide insertions or deletions, i.e., this refers to the addition of one or more nucleotides (insertions or additions) or the loss of one or more nucleotides (deletions) having as a consequence changes in the reading frame, a reading error occurring during the translation ranging from the formation of non-functional proteins to the absence of said protein.

As it is used herein, *"pol"* is understood as the gene encoding the viral enzymes necessary for the viral replication process: protease (PRO), reverse transcriptase (RT) and integrase (INT). In a particular embodiment, the sequence of the RT protein is that shown in SEQ ID NO: 2.

In a particular embodiment, the mutation in the pol gene comprises the complete or partial deletion of said gene.

As it is used herein, "complete deletion" of the sequence of a gene refers to the loss of 100% of the nucleotides forming the nucleotide sequence of said gene.

As it is used herein, "partial deletion" of the sequence of a gene refers to the loss of at least 0.5%, 1%, 5%, 10%, 20%, 40%, 50%, 60%, 70%, 80%, 90% or 99% of the nucleotides forming the nucleotide sequence of said gene.

In another particular embodiment, the mutation in the *pol* gene gives rise to a non-functional RT protein.

As it is used herein, "non-functional RT protein" refers to fact that the RT protein does not have reverse transcriptase function, i.e., it is not capable of synthesizing doublestranded DNA using single-stranded RNA as a template. Different methods of the state of the art can be used to analyze the function of a protein; said methods will depend on the function of said protein. Thus, for example, to determine whether the RT protein is functional, different assays can be performed, among others, reverse transcription reactions of an RNA sequence or analyzing the absence of replication of a virus as a consequence of having a non-functional RT protein by colorimetrically or fluorimetrically determining the presence of a marker gene of the viral genome in cells infected with a virion comprising the sequence encoding the RT protein to be analyzed.

In another particular embodiment, the viral vector of the invention comprises a genome in which said genome further comprises a second mutation in a gene different from the *pol* gene, wherein said second mutation comprises the deletion of a region comprising all or part of said gene different from the pol gene.

In another additional particular embodiment, the gene different from the *pol* gene is selected from the *env, gag, nef, tat, rev, vif, vpu* and *vpr* HIV genes.

As it is used herein, *"env"* gene is understood as the gene encoding the gp160 protein of the viral envelope. In a particular embodiment, the sequence of the gp160 protein is that shown in SEQ ID NO: 3.

As it is used herein, *"gag"* gene is understood as the gene encoding the p55 protein of the capsid formed by 3 protein subunits (MA, CA and NC). In a particular embodiment, the sequence of the p55 protein is that shown in SEQ ID NO: 4.

As it is used herein, *"nef"* gene is understood as the gene encoding the Nef protein which negatively controls CD4 and HLA molecules of the infected cell and plays a role in the pathogenicity of the virus. In a particular embodiment, the sequence of the Nef protein is that shown in SEQ ID NO: 5.

As it is used herein, *"tat"* gene is understood as the gene encoding the Tat protein, a viral messenger RNA transactivator and elongator. In a particular embodiment, the sequence of the Tat protein is that shown in SEQ ID NO: 6.

As it is used herein, *"rev"* gene is understood as the gene encoding the Rev protein responsible for processing messenger RNA and transporting it to the cytoplasm. In a particular embodiment, the sequence of the Rev protein is that shown in SEQ ID NO: 7.

As it is used herein, *"vif"* gene is understood as the gene encoding the Vif protein (SEQ ID NO: 8) associated with the infectivity of the extracellular virions. In a particular embodiment, the sequence of the Vif protein is that shown in SEQ ID NO: 8.

As it is used herein, *"vpu"* gene is understood as the gene encoding the Vpu protein involved in the release of the virions. In a particular embodiment, the sequence of the Vpu protein is that shown in SEQ ID NO: 9.

As it is used herein, *"vpr"* gene is understood as the gene encoding the Vpr protein which acts as an accelerator for the replication cycle at different levels. In a particular embodiment, the sequence of the Vpr protein is that shown in SEQ ID NO: 10.

In an additional particular embodiment, the viral vector of the invention further comprises a marker gene, wherein said marker gene is located at the site occupied by the region deleted by means of said second mutation.

As it is used herein, "marker gene" or "reporter gene" is understood as a gene the product of which gives rise to a signal that can be readily measured or detected. Non-limiting illustrative examples of marker genes suitable for carrying out the present invention include, among others, GFP (green fluorescent protein) gene, *LacZ* gene encoding the β-galactosidase protein, the Renilla or firefly luciferase gene encoding the luciferase protein, etc.

In another particular embodiment, the viral vector of the invention further comprises a sequence, originating from a second HIV genome, homologous to that of the region deleted by means of said second mutation, wherein the genome of said second HIV is different from the HIV genome containing the region deleted.

As it is used herein, "homologous sequence" refers to the sequence of a gene of a genome A equivalent to a sequence of that same gene in a genome B; i.e., by way of illustration, for the sequence of the env gene in a genome A (e.g., an HIV genome), its homologous sequence will be the sequence of the *env* gene of a genome B (e.g., a different HIV genome).

In a particular embodiment, the genome of the second HIV originates from an HIV-infected subject.

The homologous sequences originating from the HIV genome of a subject can be obtained by means of different methods known in the state of the art, among others, from blood cells of the HIV-infected patient by means of the QIAmp DNA blood kit (Qiagen), and the viral RNA can be obtained from the plasma of an HIV-infected patient by means of the QIAmp kit (QIagen). Said extracted HIV RNA is used as a template for obtaining cDNA, using techniques known by a person skilled in the art.

In another aspect the invention relates to a non-replicative virion, hereinafter non-replicative virion of the invention, comprising a viral vector of the invention.

As it is used herein, "virion" is understood as a replication-deficient infectious viral particle comprising the viral genome packed in a capsid and, optionally, in a lipid envelope surrounding the capsid. As it is used herein, "non-replicative virion" refers to a virion lacking capacity for multiplying its genetic material and therefore new copies of said virion cannot be formed.

For the production of the virions of the invention, it is necessary to first incorporate the viral vector of the invention in a cell by using any of the transfection methods known by the person skilled in the art (see sections 9.1 to 9.5 in Ausubel, F.M. et al., Current Protocols in Molecular Biology, John Wiley & Sons Inc; ringbou edition, 2003). Non-limiting illustrative examples of transfection methods include co-precipitation with calcium phosphate, DEAE-dextran, polybrene, electroporation, microinjection, liposome-mediated fusion, lipofection, retrovirus infection and biolistic transfection. In a particular embodiment, the transfection is carried out by means of co-precipitation with calcium phosphate. As a person skilled in the art will understand, the cell in which the vector of the invention is incorporated must be capable of expressing the information encoded in said viral vector of the invention and packing the proteins of the virus and the genetic material thereof to form the viral particles. Cells suitable for carrying out the present invention include, among others, cells from the 293T cell line, a cell line derived from the highly transfectable 293 cell line (derived from human embryonic kidney cells), to which cell the SV40 T antigen of SV40 has been inserted, available in the American Type Culture Collection (ATCC). The virions produced are released into the culture medium of the cells transfected with the vectors of the invention, therefore to obtain the virions of the invention it is necessary to collect the supernatants after several hours post-transfection, preferably 24 and 48 hours post-transfection. The non-replicative virions of the invention can be stored at - 80°C.

In an additional embodiment, the invention relates to a method for obtaining an activated immune cell comprising the steps of:
a) contacting an immune cell with a non-replicative virion of the invention,
b) maintaining the mixture obtained in step a) in conditions suitable for infecting said immune cells with the virion and for processing the antigens originating from the non-replicative virion, and
c) optionally, recovering the activated immune cells.

As it is used herein, "activated immune cell" hereinafter activated immune cell of the invention, is understood as an immune cell which, after being treated with immunogenic factors, is capable of generating an immune response against said immunogens. Said immune cells can be, among others, lymphocytes or dendritic cells (DC).

The suitable conditions for infecting the immune cells with the virion of the invention and the processing of antigens are known by a person skilled in the art. By way of illustration, it can be carried out, for example, by maintaining 5x10⁶ immature monocyte-derived dendritic cells (MD-DC) in contact with 1x10⁹ virions for 3 hours at 37°C.

The recovery of the activated immune cells of the invention can be carried out by means of different methods known by a person skilled in the art. By way of non-limiting illustration, the recovery of said cells can be performed by means of detaching the cells adhered on the culture plate, for example, by means of using trypsin-EDTA (0.25% and 0.02%, respectively) and subsequent centrifugation at 650 g for 4 minutes at room temperature.

In another aspect, the invention relates to an *in vitro* method for obtaining a customized HIV vaccine for an HIV-infected subject, wherein said vaccine comprises activated immune cells, which comprises:
a) contacting immune cells *in vitro* with a non-replicative virion comprising a viral vector of the invention which further comprises a second mutation in a gene different from the *pol gene,* wherein said second mutation comprises the deletion of a region comprising all or part of said gene different from the *pol* gene, wherein said viral vector further comprises a sequence, originating from a second HIV genome, homologous to that of the region deleted by means of said second mutation, wherein the genome of said second HIV is different from the HIV genome containing the deleted region and in which the genome of said second HIV originates from an HIV of said HIV-infected subject;
b) maintaining the mixture obtained in step a) in conditions suitable for infecting said cells with the virion and for processing the antigens originating from the non-replicative virion;
c) detecting and quantifying the activation of the immune cells activated according to steps a) and b);
d) comparing the activation of said immune cells activated according to steps a) and b) with the activation of control immune cells, selecting the virions which cause a greater activation of the immune cells activated according to steps a) and b) than the activation of the control immune cells,
e) selecting the virions which cause a greater activation of the immune cells activated according to steps a) and b) than the activation of the control immune cells,
f) infecting immune cells of said HIV-infected subject with the non-replicative virions selected in step e), and
g) recovering the immune cells activated according to step f) .

A person skilled in the art knows how to perform the different steps comprised in the *in vitro* method for obtaining a customized HIV vaccine for an HIV-infected subject. Said method first comprises contacting immune cells *in vitro* with a non-replicative virion of the invention. In a particular embodiment, said immune cells are lymphocytes, peripheral blood mononuclear cells or monocyte-derived dendritic cells. The immune cells and the non-replicative virion of the invention must be maintained in suitable conditions so that said cells are infected by the virion and so that the antigens originating from the non-replicative virion of the invention are processed. In a particular embodiment, said conditions are 37°C for 7 days.

It is then necessary to compare the activation of said immune cells with the activation of control immune cells. Different techniques are known in the state of the art for analyzing the activation of immune cells. Non-limiting, illustrative examples are, among others, the detection of cytokine production, the detection of the number of IFN_{Y} and IL-2 producing cells, or the frequency analysis of dividing antigen-specific CD4 and CD8 cells by means of flow cytometry analysis. The virions causing an activation of the activated immune cells greater than the activation of the control immune cells are then selected and said non-replicative virions are used for infecting immune cells of said HIV-infected subject. Finally, the method comprises recovering said activated immune cells.

As used herein, "control immune cells" are understood as immune cells activated with other viral preparations, such as viruses inactivated by means of heat or chemical treatment, or heterologous gene-carrying defective viral particles, immature virions obtained by different mechanisms such as treatment with protease inhibitors or by mutations, recombinant proteins or peptide combinations.

In another aspect, the invention relates to an *in vitro* method for obtaining a customized HIV vaccine for an HIV-infected subject, wherein said vaccine comprises activated immune cells, which comprises:
a)contacting immune cells originating from said HIV-infected subject *in vitro* with a non-replicative virion comprising a viral vector of the invention which further comprises a second mutation in a gene different from the *pol* gene, wherein said second mutation comprises the deletion of a region comprising all or part of said gene different from the *pol* gene, wherein said viral vector further comprises a sequence, originating from a second HIV genome, homologous to that of the region deleted by means of said second mutation, wherein the genome of said second HIV is different from the HIV genome containing the deleted region and in which the genome of said second HIV originates from an HIV of said HIV-infected subject;
b)maintaining the mixture obtained in step a) in conditions suitable for infecting said immune cells with the virion and for processing the antigens originating from the non-replicative virion; and c)recovering the immune cells activated according to step b).

In a particular embodiment, the immune cell is selected from a lymphocyte, a peripheral blood mononuclear cell and a monocyte-derived dendritic cell.

In another particular embodiment, the method for obtaining a customized HIV vaccine further comprises the step of subjecting the activated immune cells to a treatment to ensure the safety of said activated immune cells.

"Treatment to ensure the safety" refers to those treatments which prevent the virions of the invention used in generating a customized HIV vaccine, despite being non-replicative, from being able to induce the development of AIDS in the vaccinated subject. To that end, different methods known in the state of the art can be used, including treatment with HIV RT protein and/or protease and/or integrase inhibitors and/or the inactivation of the virions with AT-2 or heat.

RT protein inhibitors suitable for use in the present invention are, among others, zidovudine (AZT), Retrovir®, didanosine (ddI), Videx®, zalcitabine (ddC), Hivid®, stavudine (d4T), Zerit®, lamivudine (3TC), Epivir®, abacavir (ABC), Ziagen®, emtricitabine (FTC), Emtriva®, tenofovir DF (TDF), Viread®, nevirapine (NVP), Viramune®, delavirdine (DLV), Rescriptor®, efavirenz (EFV), Sustiva®, etravirine (ETV), Intelence® and suitable HIV integrase inhibitors like Raltegravir (RTG) Isentress®. The inactivation of the virions of the invention by heat can be carried out at 56°C for 60 minutes. The inactivation with AT-2 (aldrithiol-2) can be carried out by means of incubating the virions with AT-2 at a concentration of 1 mM overnight at 4°C. The inactivation with protease inhibitors is performed by treating the virus-producing 293T cells while changing medium after transfection with drugs like saquinavir (SQV), Invirase®, indinavir (IDV), Crixivan®, ritonavir (RTV), Norvir®, nelfinavir (NFV), Viracept®, fosamprenavir (FOSAPV), Telzir®, lopinavir / rtv (LPV/r)-Kaletra®, atazanavir (ATV), Reyataz®, tipranavir (TPV), Aptivus®, darunavir (DRV), Prezista® at concentrations capable of inhibiting 90% of the viral maturation.

In another aspect, the invention relates to the activated immune cells obtained according to the methods of the invention.

In another aspect, the invention relates to the use of a non-replicative virion of the invention or of the activated immune cell of the invention in medicine.

In another aspect, the invention relates to the non-replicative virion of the invention or the activated immune cell of the invention for use thereof as a medicament.

The amount of virions of the invention and of activated immune cells of the invention, as well as the administration time, will be within the capacity of the person skilled in the art having the benefit of the present teaching. In fact, the administration of therapeutically effective amounts of virions of the invention and of activated immune cells of the invention can be achieved by means of a single administration, such as, for example, a single injection of a sufficient number of virions or cells to provide a therapeutic benefit to the patient who is subjected to such treatment. Alternatively, in some circumstances, it may be desirable to provide multiple or consecutive administrations, either for a relatively short or a relatively prolonged time period, which may be determined by the doctor supervising the administration of such compositions. In fact, in certain embodiments, it may be desirable to administer two or more compositions of different virions or cells of the invention either alone or in combination with one or more drugs to achieve the desired effects of the particular therapeutic regimen.

In another aspect, the invention relates to the use of a non-replicative virion of the invention or of the activated immune cell of the invention in the preparation of an HIV vaccine.

The vaccines of the invention will be administered in a pharmacologically effective amount producing an improvement of one or more symptoms of a viral disorder or preventing the progression of a viral disease or causing a regression of the disease or reducing the viral transmission. For example, a therapeutically effective amount preferably relates to the amount of a therapeutic agent which reduces the transmission index, reduces the HIV viral load or reduces the number of infected cells by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or even more. A therapeutically effective amount referring to an HIV also relates to the amount of a therapeutic agent which increases the CD4+ cell count, increases the time to progression to AIDS or increases the survival time by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or even more.

The administration of the virions of the invention or of the immune cells of the invention can be performed together with a pharmaceutically acceptable carrier or support. Occasionally, said pharmaceutically acceptable carrier or support is an adjuvant; an aluminium salt or an oil in water emulsion; an emulsifying agent and a metabolizable oil; or an immunostimulating agent, and the composition is administered to the subject by means of injection.

Said vaccines may further comprise at least one adjuvant which increases and/or mediates an immune response chosen from an innate immune response and/or an adaptive immune response. The adjuvants which can be used can increase the immune response by activating the antigen presenting cells (APC), macrophages and/or by stimulating specific groups of lymphocytes.

An adjuvant that may be suitable for the present invention can be any ligand suitable for the activation of a pathogen recognition receptor (PRR) expressed in and on dendritic cells (DC), T cells, B cells or other antigen presenting cells. The ligands activating the nucleotide binding oligomerization domain (NOD) receptor pathway may be suitable for the purposes of the invention. The adjuvants suitable for these ligands may be derived from muramyl dipeptide. The ligands activating the toll-like receptors (TLR) may also be suitable for the purposes of the invention. These receptors are members of the PRR family and are widely expressed in a variety of innate immune cells including DC, macrophages, mast cells and neutrophils.

The following can be mentioned as examples of ligands which activate TLR: for TLR4, monophosphoryl lipid A, 3-0-deacetylated monophosphoryl lipid A, *E. coli* LPS, taxol, RSV fusion protein and host heat shock proteins 60 and 70; for TLR2, lipopeptides such as N-palmitoyl-S-2,3(bispalmitoyloxy)-propyl-cysteinyl-seryl-(lysyl)3-lysine, *S. aureus* peptidoglycan, *M. tuberculosis* lipoproteins, *S. cerevisiae* zymosan and highly purified *P. gingivalis* LPS; for TLR3, dsRNA, flagellin TLR5 and TLR7 of synthetic compounds such as imidazoquinoline; or for TLR9, certain types of CpG-rich DNA. Other adjuvants useful for the invention may be T helper epitopes.

A T helper epitope is a peptide normally derived from exogenous proteins which have undergone protein degradation and processing in the endocytic pathway of antigen presenting cells (APC). In those cells, the class II major histocompatibility complex (MHC II) is associated with those peptides in the endosomes. This complex transported to the surface of the APCs can interact with a specific T cell receptor of CD4 T lymphocytes which causes the activation thereof. As is known in the art, the response of the T cell can be Th1 and/or Th2 type, depending on the helper epitope.

An example of a Th-oriented response epitope is the pan DR helper T cell epitope (PADRE). This epitope is manipulated so that it binds to the more common high affinity HLA-DRA molecules and so that it acts as a strong immunogen. It has been shown that the PADRE HTL epitope increases the strength of vaccines designed for stimulating a cellular immune response. See Alexander J, et al., Immunol. Res. 1998; 18:79-92.

The formulation of vaccines according to the invention uses an effective amount of the antigen of interest. In other words, it will include an amount of antigen which, in combination with the adjuvant, causes the subject to produce a specific and sufficient immune response such that it confers protection to the subject against subsequent exposure to HIV. When it is used as an immunogenic composition, the formulation will contain an amount of antigen which, in combination with the adjuvant, causes the subject to produce specific antibodies that can be used for diagnostic or therapeutic purposes.

The vaccine compositions of the invention may be useful for preventing HIV-1 infection or for HIV-1 infection therapy. While all animals that may be affected with HIV-1 or its equivalents can be treated in this manner, the vaccines of the invention are particularly aimed at their preventive and therapeutic uses in human beings. More than one administration may often be required to produce the desired prophylactic or therapeutic effect; the exact protocol (dose and frequency) can be established by means of standard clinical procedures.

The vaccine formulations will contain an effective amount of the selected antigen/antigens which, when combined with the adjuvant, causes the vaccinated subject to produce a sufficient and specific immune response to the antigen. The vaccine compositions can also be used therapeutically for treating subjects already infected with HIV.

In some embodiments, it may be desirable to administer combination vaccines having a component which causes an immune response mainly against HIV strains with tropism for CCR5 receptor and a second component which causes an immune response mainly against HIV strains with tropism for CXCR4 receptor. It may also be desirable for one or both components to be formed by a mixture of antigens, such as for example, a mixture of antigens each of which causes an immune response against a particular HIV strain or group of HIV strains.

In another additional embodiment, the invention relates to the use of a non-replicative virion of the invention or to the activated immune cell of the invention in the preparation of a pharmaceutical composition for treating AIDS.

In another additional embodiment, the invention relates to a non-replicative virion of the invention or to the activated immune cell of the invention for use thereof in treating AIDS.

As it is used herein, the term "treatment" relates to the act of reverting, improving or inhibiting the evolution of the disorder or condition for which such term is applied, or one or more symptoms of such disorder or condition, as well as to prevent it from occurring, having it, or alternatively delaying the onset or recurrence of a disease, disorder or condition for which such term is applied, or one or more of the symptoms associated with a disease, disorder or condition.

The invention is illustrated below based on the following examples which are provided by way of non-limiting illustration of the scope of the invention.

### EXAMPLE 1

### Production of virions

### 1.1 Generation of viral vectors

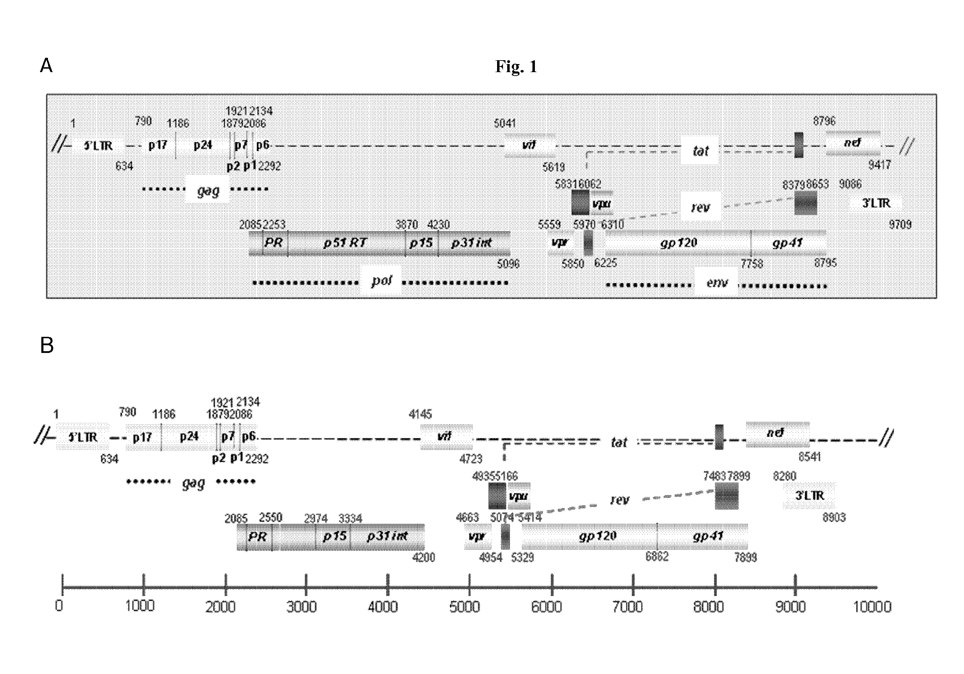

To generate the pNL4-3ΔRT vector, HIV genome-1 pNL4-3 (NIH AIDS Research & Reference Reagent Program, no. 114, accession number M19921.1 as of 1 December 2010 in the GenBank database) (SEQ ID NO: 1) (Figure 1A) was used. To that end, restriction sites which allowed readily "extracting" particular DNA fragments from the provirus matrix (such as, for example, the gene encoding RT, the *pol, gag,* nef or env genes, etc.) were initially inserted by means of directed mutagenesis in order to substitute them with the same genes originating from the isolates of patients to be assessed. This system of "cloning" and generating a "chimera virus" allows studying the characteristics of the different viral proteins of the patients. Therefore, the following main modifications were performed:
- first, the deletion of HIV-1 fragments, for example, the deletion of a fragment comprising the gene encoding reverse transcriptase (RT), specifically the fragment comprised between position 2592 and 3485 of the pNL4-3 proviral vector; and
- then, the *LacZ* gene (encoding the beta-galactosidase enzyme) was inserted substituting different sequences of the viral genome, on one hand, to find out the generation frequency of recombinant viruses, and, on the other hand, to avoid the carry-over of sequences of wild-type viruses.

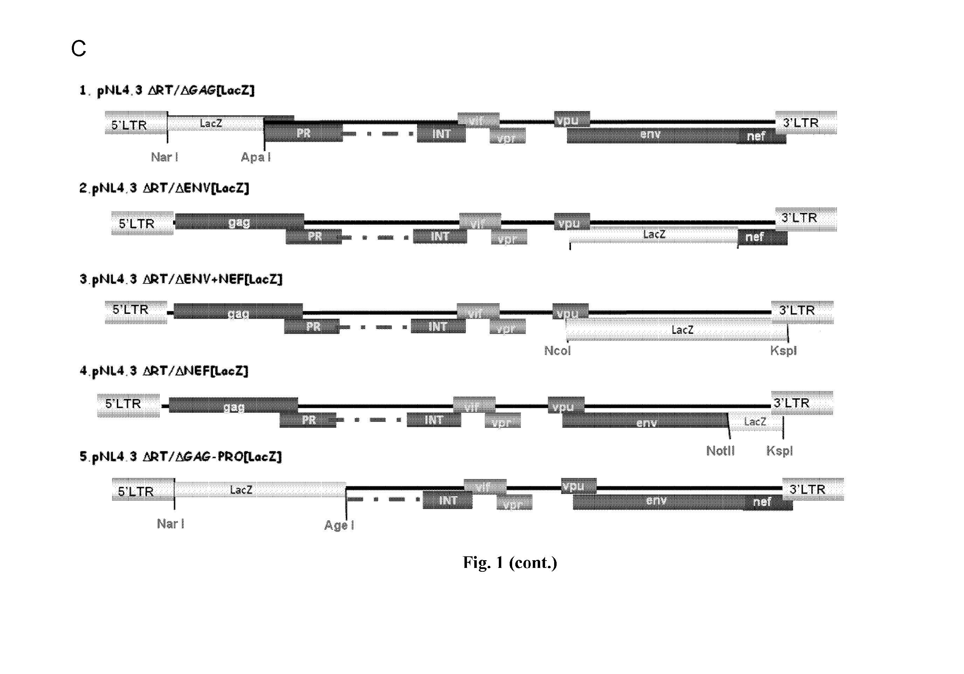

The HIV-based recombinant viral clones of the present invention are characterized in that they have the general structure depicted in Figure 1, comprising in direction 5' to 3' the following elements:
- LTR (long terminal repeats) or terminally redundant sequences containing various consensus sequences for transcription factors regulating viral expression;
- *gag,* the gene encoding the p55 protein of the capsid formed by 3 protein subunits (MA, CA and NC);
- pol, the gene encoding the viral enzymes necessary for the viral replication process: protease (PRO), reverse transcriptase (RT) and integrase (INT), and it overlaps at its 5' end with the gag element;
- *vif*, the gene encoding the Vif protein associated with the infectivity of the extracellular virions, and it overlaps at its 5' end with the pol gene and at its 3' end with *vpr;*
- *vpr,* the gene encoding the Vpr protein acting as an accelerator of the replication cycle at different levels, and it overlaps at its 5' end with the *vif* gene;
- *tat,* the gene encoding the Tat protein which is a transactivator, and its second exon is contained in the env sequence;
- *vpu*, the gene encoding the Vpu protein involved in the release of the virions;
- *env,* the gene encoding the gp160 protein of the viral envelope;
- *rev,* the gene encoding the Rev protein, responsible for processing and transporting messenger RNA to the cytoplasm, and its second exon is contained in the *env* sequence; and
- *nef,* the gene encoding the Nef protein which negatively regulates CD4 and HLA molecules of the infected cell and plays a role in the pathogenicity of the virus.

The pNL4-3ΔRT*[lacZ]*Ren viral vector described in EP1752541 as IP HIV NL LacZ/RT Ren is characterized by having the substitution of the 896 bp fragment of the *pol* gene encoding the RT between NcoI/AgeI with the *LacZ* gene. pNL4-3ΔRT[*lacZ*] was generated from this construct by means of removing the marker gene encoding the *Renilla (Ren)* luciferase protein (SEQ ID NO: 13) and restoring the sequence of the *nef* gene (90 bp) which had been previously removed, located between the NotI/XhoI restriction sites (in positions 8797 and 8887, respectively). For this purpose, the area with the oligonucleotides specific for pNL4-3, 52EU: (SEQ ID NO: 11) and 104ED: (SEQ ID NO: 12), the NotI and XhoI restriction sites being included in these oligonucleotides, was previously amplified. Once the 90 bp region of the nef gene was amplified from pNL4-3, it was cloned into the NotI (8797)/XhoI (8887) position, where the *Ren* gene had been removed, the plasmid pNL4-3 ΔRT[*lacZ*] having a size of 14,483 bp being generated.

The following step was the removal of an NarI restriction site located in the plasmid pNL4-3 outside the coding sequence of the virus, i.e., in pUC18. The previously generated plasmid (pNL4-3 ΔRT[*lacZ*]) was used and digested with the AatII and ApaI restriction enzymes, a 3613 bp fragment (insert) being obtained, where the NarI site had already been removed. This fragment was cloned with the same corresponding restriction enzymes into pNL4-3ΔRT[*lacZ*], converting it into plasmid pNL4-3 ΔRT[*LacZ*], NarI⁻. The size of the generated plasmid is about 14,483 bp.

The *LacZ* gene encoding the β-galactosidase protein (SEQ ID NO: 14) cloned into NcoI/AgeI by means of digestion with the corresponding restriction enzymes and the subsequent filling of the sticky ends with the DNA polymerase I Klenow fragment (GE Healthcare) was then removed and re-ligated, giving rise to plasmid pNL4-3 ART, NarI- hereinafter called pNL4-3 ART. The size of this plasmid generated is about 13,983 bp (Figure 1B).

Different constructs of the invention were generated from pNL4-3 ART; to that end the LacZ gene was cloned into the pNL4-3ΔRT vector in the position of gag gene (pNL4-3ΔRT/Δ*gag*-[*LacZ*]), env gene (pNL4-3ΔRT/Δ*env*-[*LacZ*] ), *env-nef* gene (pNL4-3ΔRT/Δ*env-nef*-[LacZ]), *nef* gene (pNL4-3ΔRT/Δ*nef*-[*LacZ*]) and in *gag-pro* gene (pNL4-3ΔRT/Δ*gag-pro*-[*LacZ*]) (Figure 1C, Table 1) for the purpose of increasing the cloning efficacy and avoiding the carry-over of minority populations of the reference virus. Thus, the ligated clones without the insert from the patient gave blue colonies, whereas the plasmid which incorporated the gag gene, *env* gene, *env-nef* gene, *nef* gene, and/or *gag-pro* gene originating from the patient gave white colonies.

The following viral vectors were generated from pNL4-3 ΔRT (Figure 1C, Table 1):
1. pNL4-3 ΔRT/Δ*gag* [*LacZ*] : It is characterized by the removal of part of the gag gene, 1368 bp, between the NarI (in position 637) and ApaI (in position 2006) restriction sites of pNL4-3ΔRT; the 500 bp amino-terminal end of the *LacZ* gene was cloned in its place with the same enzyme. The *LacZ* gene was amplified by means of PCR of plasmid pUC19 (Invitrogen) with the NarI-*LacZ*-UP (SEQ ID NO: 15) and ApaI-*lacZ*-D (SEQ ID NO: 16) oligonucleotides. The plasmid generated is referred to as pNL4-3 ΔRT/Δ*gag [LacZ]* with a total size of 13,115 bp.
2. pNL4-3 ΔRT KspI⁺: A new KspI (SacII) restriction site was introduced in position 9433 of pNL4-3 by directed mutagenesis. In order to generate this plasmid, the BamHI-NaeI fragment of pNL4-3-NotI was first subcloned into pBluescript II SK(-)vector (Stratagene) at the same sites, the pBluescript SK/NL BamHI-NaeI vector being generated, and on the latter directed mutagenesis was performed to **generate the KspI site with the following** oligonucleotides: M-KspI-UP (SEQ ID NO: 17) and M-KspI-Down (SEQ ID NO: 18), the plasmid pBluescript SK/NL BamHI-NaeI, KspI+, being generated; the BamHI-NaeI fragment was then extracted from the latter plasmid with KspI mutation and was cloned into pNL4-3 ΔRT at the same restriction sites, giving rise to pNL4-3 ΔRT KspI ⁺. The presence of the new target KspI restriction was confirmed by means of sequencing in the corresponding position of pNL4-3. The size of the vector generated was 13,983 bp.
3. pNL4-3 ΔRT/Δ*nef* [*LacZ*]*:* The 640 bp nef gene between NotI (nt 8797 of pNL4-3) and KspI (SacII) (nt 9437) was removed from pNL4-3 ΔRT KspI⁺ and the 500 bp amino-terminal end of the *LacZ* gene was cloned in its place with the same enzymes. The *LacZ* gene was amplified by means of PCR of plasmid pUC19 (Invitrogen) with the NotI-LacZ-UP (SEQ ID NO: 19) and KspI-LacZ-D (SEQ ID NO: 20) oligonucleotides. 13,843 bp plasmid pNL4-3 ΔRT/Δ*nef* [*LacZ*] was generated.
4. pNL4-3 ΔRT KspI⁺, NcoI⁺: A new NcoI restriction site was introduced in position 6113 in pNL4-3 by mutagenesis. The oligonucleotides used were: Mut-NcoI-Env-UP (SEQ ID NO: 21) and Mut-NcoI-Env-down (SEQ ID NO: 22). The presence of the new target NcoI restriction was confirmed by means of sequencing in the corresponding position of pNL4-3. The size is 13,838 bp.
5. pNL4-3 ΔRT/Δ*env* [*LacZ*]: The 2683 bp env gene between NcoI (nt 6113) and NotI (nt 8797) was removed from pNL4-3 ΔRT KspI⁺, NcoI⁺; the 500 bp amino-terminal end of the *LacZ* gene was cloned in its place with the same enzymes. The *LacZ* gene was amplified by means of PCR of the plasmid pUC19 (Invitrogen) with the 127 (SEQ ID NO: 23) and Not-LacZ-down (SEQ ID NO: 24) oligonucleotides. 11,800 bp plasmid pNL4-3 ΔRT/Δ*env [LacZ]* was generated.
6. pNL4-3 ΔRT/Δ*env-nef* [*LacZ*] : The 3320 bp env and *nef* genes between the NcoI and KspI (SacII) restriction sites in pNL4-3 ART, KspI⁺, NcoI⁺ were removed at the same time, the 500 bp amino-terminal end of the *LacZ.* gene was cloned in their place with the same enzymes. The *LacZ* gene was amplified by means of PCR of the plasmid pUC19 (Invitrogen) with the 127 (SEQ ID NO: 23), and KspI-LacZ-D (SEQ ID NO: 20) oligonucleotides. 11,160 bp plasmid pNL4-3 ΔRT/Δ*env-Nef* [*LacZ*] was generated.
7. pNL4-3 ART, KspI⁺, NcoI⁺, AgeI⁺: A new Age I restriction site was introduced in position 2572 according to the sequence of pNL4-3 by mutagenesis. The primers used were: Mut-AgeI-UP (SEQ ID NO: 25) and Mut-AgeI-Down (SEQ ID NO: 26). The presence of the new target AgeI restriction was confirmed by means of sequencing in the corresponding position of pNL4-3. The size is 13,838 bp.
8. pNL4-3 ΔRT/Δ*gag-pro*[*LacZ*] : The 1935 bp fragment comprising the gag and pro genes comprised between the Nar I (in position 637) and AgeI (in position 2572) sites of the pNL4-3 ART, KspI⁺, NcoI⁺, AgeI⁺ was removed; the 500 bp amino-terminal end of the *LacZ* gene was cloned in its place with the same enzymes. The *LacZ* gene was amplified by means of PCR of the plasmid pUC19 (Invitrogen) with the primers NarI-*LacZ*-UP (SEQ ID NO: 15) and AgeI-*lacZ*-D (SEQ ID NO: 27). 12,403 bp plasmid pNL4-3 ΔRT/Δ*gag-pro [LacZ]* was generated.

**Table 1**

| List of plasmids generated in the invention | | | | | |
|---|---|---|---|---|---|
| PLASMIDS | INTERMEDIATE PLASMIDS | bp | New restriction sites (position pNL4.3) | Sites removed | Enzymes for cloning |
| pNL4-3 | | 14879 | | | |
| | pNL4-3 ΔRT | 13983 | | NarI (pUC18) | |
| pNL4-3 ΔRT | | 13983 | | | |
| pNL4-3 *Δ*RT/*Δgag*[*LacZ*] | | 13115 | | | NarI + ApaI |
| | pNL4-3 ΔRT KspI + | 13983 | KspI/SacII (9433) | | |
| pNL4-3 ΔRT/Δ*nef*[*LacZ*] | | 13843 | | | NotI + KspI |
| | pNL4-3 ΔRT KspI+ NcoI+ | 13838 | NcoI (6113) | | |
| pNL4-3 ΔRT/Δ*env*[*LacZ*] | | 11800 | | | NcoI + NotI |
| pNL4-3 ΔRT/Δ*env-nef*[*LacZ*] | | 11160 | | | NcoI + KspI |
| | pNL4-3 ΔRT KspI+ NcoI+ AgeI+ | 13838 | AgeI+ (2572) | | |
| pNL4-3 ΔRT/Δ*gag*-*pro*[*LacZ*] | | 12403 | | | NarI + AgeI |

Vectors/chimeras were then constructed by means of cloning the *env, gag, gag-pro, env-* nef and/or *nef* gene fragments of HIV-1-infected patients in the previously described corresponding vectors (Figure 1C, Table 1) in order to subsequently generate virions and to analyze the induced immune response.

The total blood viral DNA of HIV-1 positive patients under study were obtained from the cell pellet by means of the QIAmp DNA blood kit (Qiagen).

For the extraction of viral RNA, 1 ml of plasma from the patients under study was used; the viral RNA was extracted by means of the QIAmp kit (QIagen). The extracted HIV-1 RNA was used as a template to obtain cDNA. The RNA was initially transcribed and subsequently amplified using the specific primers for each of the areas to be characterized.

The amplified fragments were:
1. gag fragment: 1793 bp were amplified by means of specific primers [(107 (SEQ ID NO: 28) / 122 (SEQ ID NO: 29)) and (163 (SEQ ID NO: 30) / 123 (SEQ ID NO: 31))] which include NarI and ApaI restriction sites in primers 163 and 123, respectively, necessary for the subsequent cloning thereof into the pNL4-3 ΔRT/Δ*gag*[*LacZ*] proviral vector substituting the corresponding LacZ gene.
2. gag-pro fragment: 1935 bp were amplified by means of specific primers [(107 (SEQ ID NO: 28) / 125 (SEQ ID NO: 40)) and (163 (SEQ ID NO: 30) / 159(SEQ ID NO: 41))] which include NarI and AgeI restriction sites in primers 163 (SEQ ID NO: 30) and 159 (SEQ ID NO: 4), respectively, necessary for the subsequent cloning thereof into the pNL4-3 ΔRT/Δ*gag-pro*[*LacZ*] proviral vector, the *LacZ* gene being removed first.
3. nef fragment: 637 bp were amplified by means of specific primers [(117 (SEQ ID NO: 35) / 118 (SEQ ID NO: 34) and (119 (SEQ ID NO: 32) / 121 (SEQ ID NO: 33))] which include NotI and KspI sites in primers 119 (SEQ ID NO: 32) and 121 (SEQ ID NO: 33), respectively, necessary for the subsequent cloning thereof into the pNL4-3 ΔRT/Δ*nef*[*LacZ*] proviral vector after removing the *LacZ* gene located in the position of the *nef* gene.
4. env fragment: 2683 bp were amplified by means of specific primers [(101 (SEQ ID NO: 37) / 102(SEQ ID NO: 36)) and (126 (SEQ ID NO: 39) / 161 (SEQ ID NO: 38))] which include NcoI and NotI restriction sites in primers 126 (SEQ ID NO: 39) and 161 (SEQ ID NO: 38), respectively, necessary for the subsequent cloning thereof into the pNL4-3 ΔRT/Δ*env*[*LacZ*] proviral vector, the LacZ gene being removed first.
5. env-nef fragment: 3320 bp were amplified by means of specific primers [(101 (SEQ ID NO: 37) / 118(SEQ ID NO: 34)) and (126 (SEQ ID NO: 3) / 121(SEQ ID NO: 33))] which include NcoI and KspI restriction sites in primers 126 (SEQ ID NO: 3) and 121(SEQ ID NO: 33), respectively, necessary for the subsequent cloning thereof into the pNL4-3 ΔRT/Δ*env-nef*[*LacZ*] proviral vector, the *LacZ* gene being removed first.

In any case, after amplification, an enzymatic digestion of the amplified product and of the vector was performed with the corresponding enzymes in each case (Table 1), according to the manufacturer's instructions, and an *in vitro* ligation process was then performed using T4 ligase according to the manufacturer's instructions. All the plasmids were amplified in bacterial strain stbl2^{tm} (Invitrogen) and they were subsequently purified by means of maxi-preparations with the Qiagen-tip 500 kit (QiagenTM) and were stored at -20°C. All the plasmids were verified by means of restriction analysis and subsequent sequencing.

### 1.2 Production and concentration of virions

Virions were generated by means of transfecting 293T cells (ATCC) with the different viral vectors under study through the calcium phosphate method (Profection Kit; Promega). 5 µg of plasmid DNA from different viral vectors (Qiagen, Valencia, CA) were used. The day before transfection, 1.5x10⁶ cells were seeded in 10 ml of DMEM-10 (DMEM supplemented with 10% (v/v) fetal bovine serum) in 75 cm² culture bottles. At 24 hours post-transfection, the medium was changed and at 48 hours the supernatants were collected, clarified and subsequently concentrated. Once the virions were obtained, they were stored at -80°C. In any case, a supernatant was obtained in cells treated in the same manner but transfected with the "empty vector" which was used as negative control. The supernatant collected after transfection was centrifuged in 30 ml threaded tubes for 32 minutes at 28,000 rpm in a Thermo scientific Sorvall® WX Ultra Centrifuge Series 80 centrifuge (Thermo Scientific Corporation). The supernatant was discarded. The pellet obtained after centrifugation was resuspended in 500 µl of phosphate buffered saline (PBS) which was transferred to an Eppendorf tube. They were again centrifuged for 10 minutes at 40,000 rpm. The supernatant was discarded and the pellet was resuspended in 500 µl of PBS.

The detection of the p24 antigen by means of the ELISA/Ag HIV technique was used to quantify the viral immunogen (Innogenetics, Belgium). The quantification was always performed before and after its concentration.

### EXAMPLE 2

### Characterization of the virions

### 2.1 Assessment of the viral replication in MT-2 cells

The assessment of the viral replication was performed by means of using marker gene-carrying virions, specifically the *Ren* (renilla) gene. The assessment of the viral replication was analyzed by means of an assay in MT-2 cells obtained from NIH AIDS Research and Reference Reagent Program, Catalog number: 237. The virions were obtained by transfection with the corresponding pNL4-3 Ren WT or pNL4-3 ΔRT Ren viral vectors, described in EP1752541, in 293T cells. At 48 hours the supernatant was collected and the p24 antigen was quantified by capture ELISA [Innogenetics, Belgium]. Part of the NL4-3 Ren WT virions was heat-inactivated [56°C for 60 minutes)].

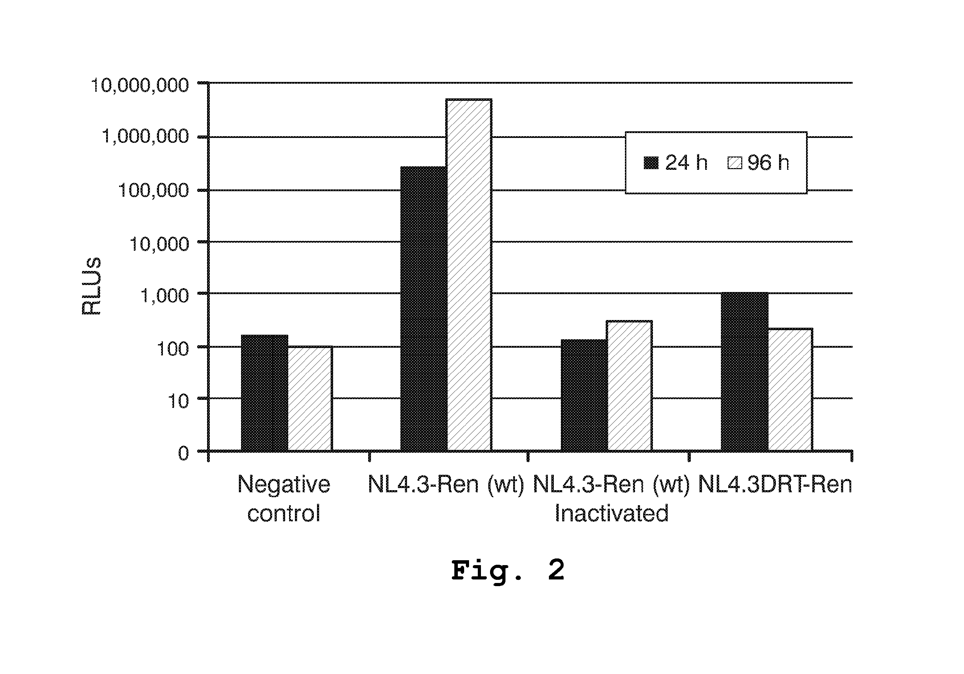

In order to assess the replication capacity, 5 x 10⁴ MT-2 cells were seeded per well in 96-well round bottom plates at a final volume of 200 µl. They were subsequently infected with different dilutions of the viral stocks (NL4-3 Ren WT, heat-inactivated NL4-3 Ren WT or NL4-3 ΔRT Ren) which had previously been quantified by means of p24 antigen quantification. The assay was performed in triplicate. The infections were maintained at 37°C in 5% CO₂ atmosphere. At 24 and 96 hours of the infection, the luciferase activity was determined with the Renilla Luciferase Assay System (Promega) system following the manufacturer's recommendations for Renilla. The cells were lysed in 100 µl of the lysis buffer for 5 minutes at 4°C. The luciferase activity of these cell extracts was evaluated by introducing 50 µl of the cell lysate in a plate illuminator (Orion, Berthold) programmed for injecting 50 ml of substrate. This value provides the relative light units (RLUs) per ml of viral stock viral at 24 or 96 hours of infection in MT-2 cells (Table 2), Figure 2.

As can be seen in Table 2, both the heat-inactivated NL4-3 Ren WT virions and the NL4-3 ΔRT Ren virions lack replicative capacity.

**Table 2**

| Quantification of luciferase activity to analyze viral infection/replication in MT-2 cells, 24 and 96 hours post-transfection | | |
|---|---|---|
| | 24 hours | 96 hours |
| Negative control | 143 RLUs | 100 RLUs |
| NL4-3 Ren WT | 261841 RLUs | 5x10⁶ RLUs |
| Heat-inactivated | 129 RLUs | 400 RLUs |
| NL4-3 Ren WT | | |
| NL4-3 ΔRT Ren | 922 RLUs | 200 RLUs |

| | | |
|---|---|---|
| RLUs: Relative light units | | |

### 2.2 Assessment of the replication of NL4-3 ΔRT virions in TZM-bl cells

The replication of the NL3.4 ΔRT (DRT) and NL4-3 (WT) virions was assessed by means of quantifying luciferase activity in TZM-bl cells.

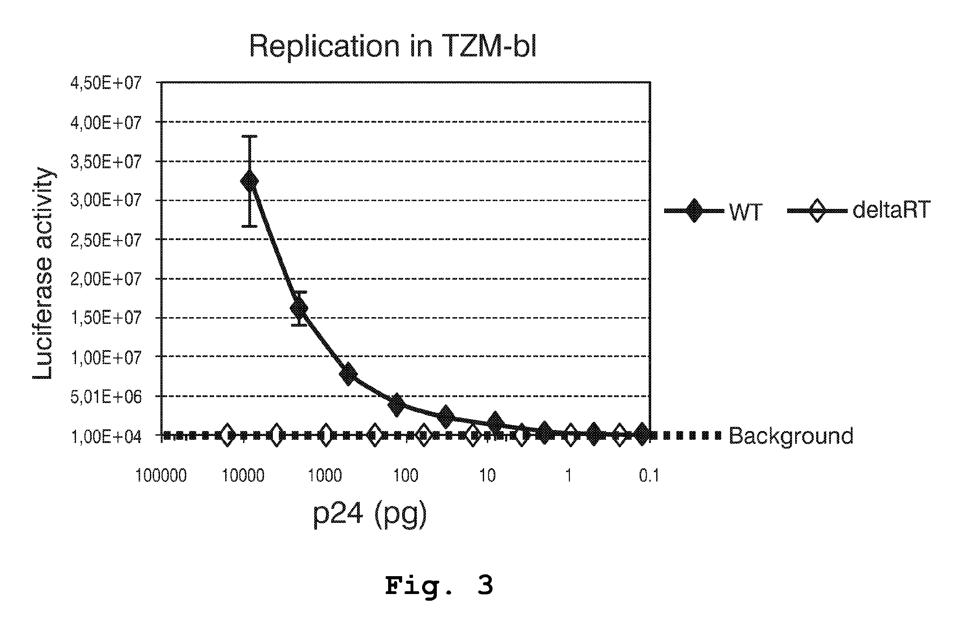

The TZM-bl indicator cells express the CD4 receptor and the CCR5/CXCR4 co-receptors with an integrated LTR-Luc reporter system. After assessing the p24 antigen, 1:4 dilutions of the transfection supernatant where the virions are placed in 96-well plates were prepared in triplicate. 10,000 TZM-bl cells were added and the plate was maintained in an incubator at 37°C in 5% CO₂ atmosphere. After 72 hours, the luciferase activity present in the cell extracts was analyzed in a luminometer (Turner Biosystems, Sunnyvale, CA) using the Biotherma assay kit (Sweden) following the manufacturer's instructions. As can be seen in Figure 3, the NL4-3 ΔRT (DRT) virions lacked replicative capacity.

### 2.3 Confocal microscopy assay

### A) Transfections in 293T cells

293T cells were co-transfected with the plasmids: pGAG-GFP (5 µg), pNL4-3 WT (3µg) + pGAG-GFP (2 µg) and pNL4-3 ΔRT (3µg) + pGAG-GFP (2 µg). At 48 hours post-transfection, the supernatant was collected and used to subsequently infect new susceptible cells. The cells were fixed with FACS lysis solution for 20 minutes at room temperature. After washing with PBT (PBS, 0.1% Tween® 20) the cells were stained with 4' ,6-diamidino-2-phenylindole (DAPI) (1/10000) and phalloidin (1/1000) for 30 minutes at room temperature. Subsequently, the cells were mounted with mounting medium (Vectashield) (Figure 4 A).

### B) Infection

The virion was quantified by means of assessing the p24 antigen by ELISA (Innogenetics, Belgium). The PBMC cells (peripheral blood mononuclear cells) (25x10⁶) were infected with the amount of virions corresponding to 2 µg of p24 antigen of the following virions obtained by co-transfection in 293T cells: pNL4-3 + pGAG-GFP, pNL4-3 ΔRT + pGAG-GFP and 1 ml of GAG-GFP.

It was incubated between 3-4 hours at 37°C. The cells were subsequently collected, fixed, stained and prepared for visualization by means of confocal microscopy following the same process as that for the transfected cells. The images were taken with a LEICA SP2 confocal microscope and processed with ImageJ (Figure 4B).

Both the virions originating from the pNL4-3WT + pGAG-GFP co-transfection and pNL4-3 ΔRT + pGAG-GFP were capable of infecting PBMCs.

### 2.4 Visualization of the virion by means of electron microscopy

293T cells were transfected using the mammal transfection kit (Promega) with two different pNL4-3 (5 µg) and pNL4-3 ΔRT (5 µg) constructs. 48 hours after transfection, the supernatant was removed and the cells were cleaned with 1X PBS. The PBS was removed and the cell monolayer was maintained in 10 ml of 2.5% glutaraldehyde in PB (phosphate solution) for 20 minutes at 4°C. After these 20 minutes, the cells were passed to a 15 ml falcon and were centrifuged for 5 minutes at 1500 rpm (C. Lopez-Iglesias et al., Ultrastruct Mol Struct Res. 1988; 101 (1): 75-91). The supernatants were clarified and subsequently concentrated by centrifugation for 32 minutes at 28,000 rpm in a Thermo scientific Sorvall® WX Ultra Centrifuge Series 80 centrifuge (Thermo Scientific Corporation). Sorvall Ultra Series. The supernatant was discarded and the sediment obtained after centrifugation was resuspended in 500 µl of PBS. It was again centrifuged for 10 minutes at 40,000 rpm. The sediment was resuspended in 500 µl of 2.5% glutaraldehyde in PB. Once the sediment was properly resuspended, it was left for 30 minutes at 4°C with the glutaraldehyde and after that time the glutaraldehyde was changed for another 500 µl of new glutaraldehyde. It was left for 1 hour at 4°C, the glutaraldehyde was removed and washed two or three times with 1% PB and it was left in PB until it was used in electron microscopy.

The protocol described by Lopez-Iglesias *et al.* (C. Lopez-Iglesias et al., Ultrastruct Mol Struct Res. 1988; 101 (1): 75-91) was used for staining the samples and for the inclusion in paraffin.

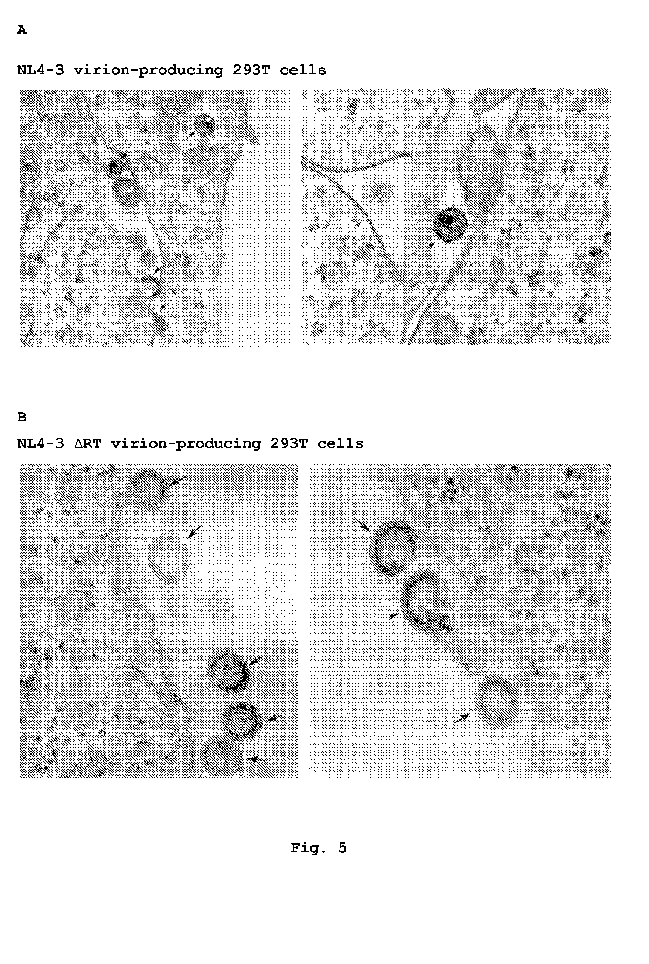

As can be seen in Figure 5A, the NL4-3 viral particles were perfectly formed (arrows) and their core well defined, and gemmating viral particles were also observed. In contrast, the NL4-3 ΔRT virions were immature and neither the formed core (arrows) nor the typical compaction of a WT viral particle could be observed in Figure 5B.

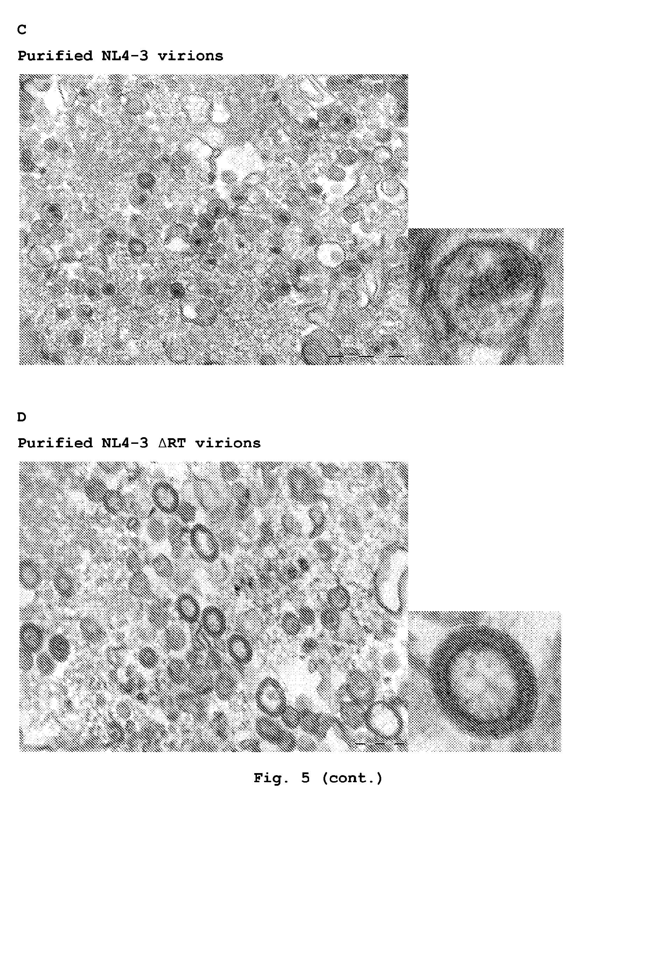

It can be observed in Figure 5C that the NL4-3 viral particles were perfectly formed in 90% of the cases although 10% defective, immature particles are observed. The purified viral particles obtained from transfecting the 293T cells transfected with the pNL4-3 ΔRT constructs can be observed in Figure 5D. It can be observed that 93% of the population has immature viral particle morphology, no mature particles being observed.

### 2.5 Analysis of the expression of the HIV-1 gag protein by means of western-blot (wb)

In order to analyze the expression of the HIV-1 gag protein, extracts from 293T cells and from purified virions obtained 48 hours post-transfection in 293T cells with the indicated plasmids (pNL4-3 and pNL4-3 ART) were used. Approximately 2 x 10⁶ 293T cells per experimental point collected at 48 hours of transfection were used for preparing the cell extracts. The cells were washed in PBS and in buffer A (10 mM Hepes pH 8, 10 mM KCl, 15 mM MgCl₂ and 1 mM DTT), resuspended in 20 µl of buffer C (20 mM Hepes pH 8, 0.1% NP-40, 25% glycerol, 420 mM NaCl, 10 mM KCl, 1.5 mM MgCl₂, 0.2 mM EDTA (ethylenediaminetetraacetic acid), 0.5 µg/ml leupeptin, 0.5 µg/ml pepstatin, 0.5 µg/ml aprotinin, 1 mM PMSF (phenylmethylsulfonyl fluoride) and 1 mM DTT (dithiothreitol)), left to lyse for 15 minutes at 4°C and centrifuged at 4,062 g for 10 minutes at 4°C. The supernatant obtained was diluted in buffer D (20 mM Hepes pH 8, 20% glycerol, 50 mM KCl, 0.2 mM EDTA, 0.5 µg/ml leupeptin, 0.5 µg/ml pepstatin, 0.5 µg/ml aprotinin, 1 mM PMSF and 1 mM DTT) depending on the desired final protein concentration.

The assessment of protein concentration was determined by means of the Bradford reaction (Biorad) (Bradford MM, Anal Biochem 1976; 72:248-54).

10 µg of the different extracts were migrated in 10% polyacrylamide-SDS (sodium dodecyl sulfate) gel to perform the Western-blot (WB). The transfer of the gels to polyvinylidene fluoride (PVDF) membranes (Sigma-Aldrich) previously incubated for 5 minutes in methanol was performed in transfer buffer (20 mM glycine, 150 mM Tris-base and 20% methanol) using an electrotransfer apparatus (Pharmacia). The membranes were blocked by incubation at 37°C for 1 hour with 5% milk powder in 0.1% PBS/Tween-20. After blocking, the membranes were incubated with a monoclonal antibody directed against the p24 protein (clone 491, Santa Cruz Laboratories) at a 1:1000 dilution. After three 5-minute washings with 0.1% PBS/Tween-20, they were incubated with peroxidase-bound mouse anti-immunoglobulin antibodies for one hour at 37°C. Three 5 minute-washings were subsequently performed with 0.1% PBS/Tween-20. The detection of the antigen-antibody interactions was performed by means of the SuperSignal WestPico Chemiluminescent Substrate (Pierce) chemiluminescence system and exposure to photographic film (Hyperfilm ECL, GE HEALTHCARE).

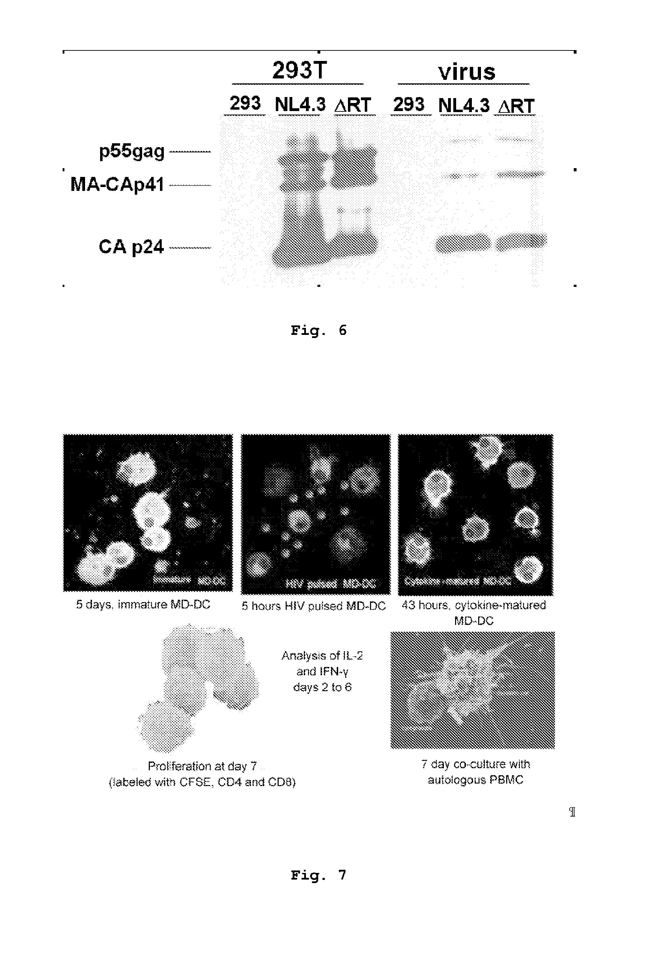

As seen in Figure 6, both the NL4-3 and NL4-3 ΔRT (ART) virions produced the gag protein.

### EXAMPLE 3

### Assessment of the cellular immune response

Once the NL4-3 ΔRT virions have been obtained and characterized, the cellular immune response was assessed in two ways. It was first assessed by means of an ex *vivo* model with monocyte-derived dendritic cells (MD-DC), the capacity for producing the specific anti-HIV cellular response in CD4+ and CD8+ cells was assessed. Assays were also performed with PBMC to detect the specific anti-HIV cellular response, among others, intracellular cytokine detection (ICS) and the assessment of the number of IFN-γ producing cells and by means of ELISPOT.

### 3.1 Ex vivo generation of monocyte-derived dendritic cells (MD-DC) pulsed with immunogen

The autologous MD-DCs were obtained from the monocytes present in the PBMC obtained from an anticoagulated sample. The PBMCs were washed three times in serum-free culture medium, X-VIVO15 and resuspended (5x10⁶/ml) in said culture medium supplemented with 1% inactivated autologous serum and they were deposited in culture vials at 37°C and 1% CO₂ for 2 hours, during which time the monocytes adhered to the surface of the vial. The non-adhered cells (lymphocytes) were removed by suction. The adhered monocytes were cultured for 5 days in X-VIVO 15 supplemented with 5% inactivated autologous serum in the presence of human recombinant IL-4 and GM-CSF at the dose of 1000 IU/ml for each cytokine and with or without 1 mM of AZT to test whether or not it was better to suppress the possible viral replication in relation to patients without antiretroviral treatment. After 5 days in culture, the monocytes turned into immature dendritic cells (DC) and they were ready to be pulsed with the different immunogens. At least 5x10⁶ immature MD-DC were used for pulsing, they were washed two times in medium and resuspended in 1 ml of medium containing the inactivated HIV-1 or immunogen aliquot (1x10⁹ virions) as well as GM-CSF+ IL4 (1000 IU/ml of each), they were maintained at 37°C for 5 hours. A cocktail of human recombinant cytokines (TNF-alpha+IL-6+IL-1β) inducing the maturation of MD-DCs was then added into the culture and it was prolonged for about 40 additional hours (Figure 7). All the MD-DCs in culture (at least 5x10⁶) were collected and centrifuged, and the supernatant was aliquoted to perform the latest possible cytokine and remaining HIV-1 measurements. The MD-DCs were washed two times with X-VIVO10 culture medium.

As controls in all the experiments, the MD-DCs were also pulsed with HIV recombinant proteins (p24), as positive control, HIV_1 recombinant p24 (Protein Sciences Corp. Meridien, CT, USA). They were also pulsed with the SEA superantigen as positive control. The same steps were repeated for the negative controls with the exception that the MD-DCs were not pulsed with the immunogen.

### 3.2 Measurement and analysis of the immune response

### 3.2.1 Labeling with CFSE and flow cytometry analysis

The PBMCs were labeled for 20 minutes at 37°C with a 1.5 mM C F S E (5-6-carboxyfluorescein diacetate succinimidyl ester) solution. After 2 washings, the labeled PBMCs were stimulated with HIV-1 recombinant proteins and the relevant controls for 6 days in culture medium supplemented with 10% fetal calf serum (FCS) in an incubator at 37°C and 5% CO₂. Subsequently and after the corresponding surface labeling, the frequency of CD4 and CD8 antigen specific cells that had been divided for each stimulus was evaluated by means of flow cytometry analysis (Figure 7).

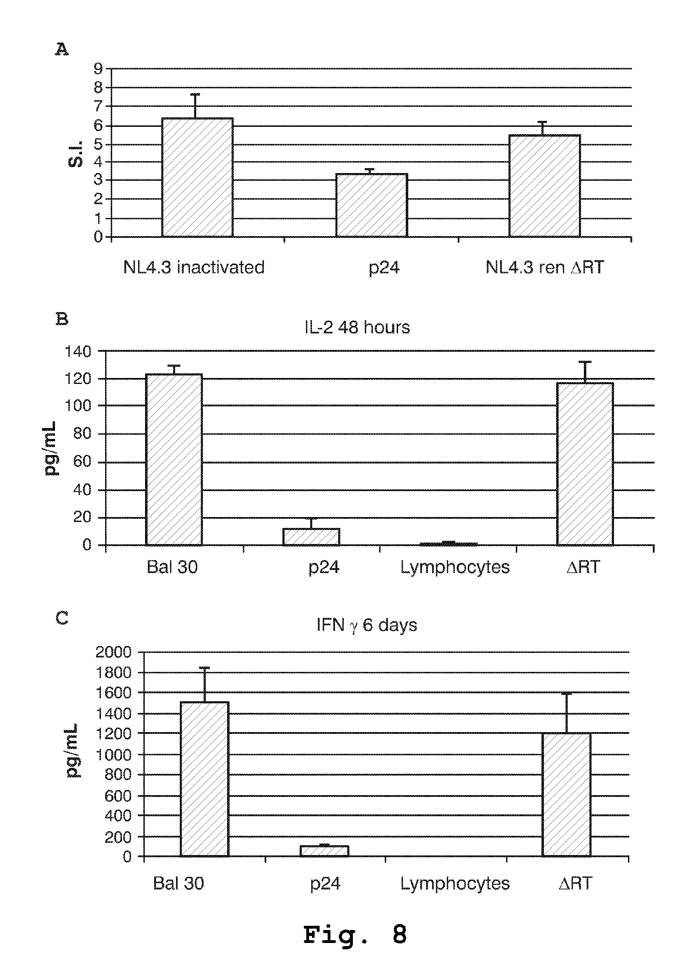

The dilution of the CSFE label was also evaluated after co-culturing for 2 days MD-DCs pulsed with the different types of immunogens and fresh autologous T cells in a DC:T cell ratio of 1:10. *In vitro* studies were performed by pulsing MD-DC cells with NL4-3 ΔRT virions and analyzing the response induced in the autologous T cells of HIV-1 patients. The data obtained indicated that after co-culturing MD-DCs pulsed with NL4-3 ΔRT and autologous T lymphocytes, an increase of cell proliferation and cytokine production (IL-2 and IFN- ã) with respect to that obtained with recombinant proteins or with the heat-inactivated autologous virus itself (Figure 8) was generated in particular patients.

### 3.2.2 Detection of the intracellular cytokine production of specific anti-HIV-1 CD4/CD8 lymphocytes

After culturing the PBMCs of the patients for 6 or 18 hours with groups of HIV-1 peptides, recombinant virions and co-stimulating antibodies (CD28 and CD49d) in the presence of Brefeldin A (10 µg/ml), the cells were fixed and permeated. The phenotypic markers of the CD4/CD8 lymphocytes were subsequently added and the HIV-1 specific T lymphocytes were identified and quantified by means of staining with anti-IFN-γ, anti-TNF-α and anti-IL-2, and activation labels (CD38, CD69) after the subsequent flow cytometry analysis.

Similarly, the measurement of CD69 expressing and IFN-γ producing CD4 and CD8 T cells was performed by means of intracellular staining and cytofluorometric analysis after re-stimulating the autologous T lymphocytes with CDDM for 1-2 days. 3.2.3 Analysis of the specific response of CD8+ T cells by means

### of ELISPOT

The analysis of the specific response of CD8+ T cells was performed by means of using a specific ELISPOT based on the determination of the production of IFN-γ by the CD8+ T cells against dominant epitopes of the HIV-1 proteins restricted for HLA-class I antigens. The cells were stimulated with pools of 15-mer overlapping synthetic peptides of the HIV-1 gag, pol, env and nef proteins, chimeric recombinant virions.

Assays with depletion of cell subpopulations (CD4, B, NK) were also performed by means of immunomagnetic selection to analyze the degree of participation thereof in the response obtained.

### 3.3 High-resolution HLA typing

High-resolution HLA typing was evaluated by means of PCR-SBT of the genomic DNA of peripheral blood mononuclear or polynuclear cells and/or cells from B cell lines. The polymorphic exons of the genes encoding MHC (mainly exon 2 of the DRB1 gene) were amplified using generic primers. The PCR products were sequenced by using the automatic fluorescent sequencing method and the sequences obtained were analyzed by means of suitable software (Perkin-Elmer).

### 3.4 Optimization of the ex vivo assay for the detection and characterization of the immune responses against HIV-1 using defective recombinant and non-replicative virions

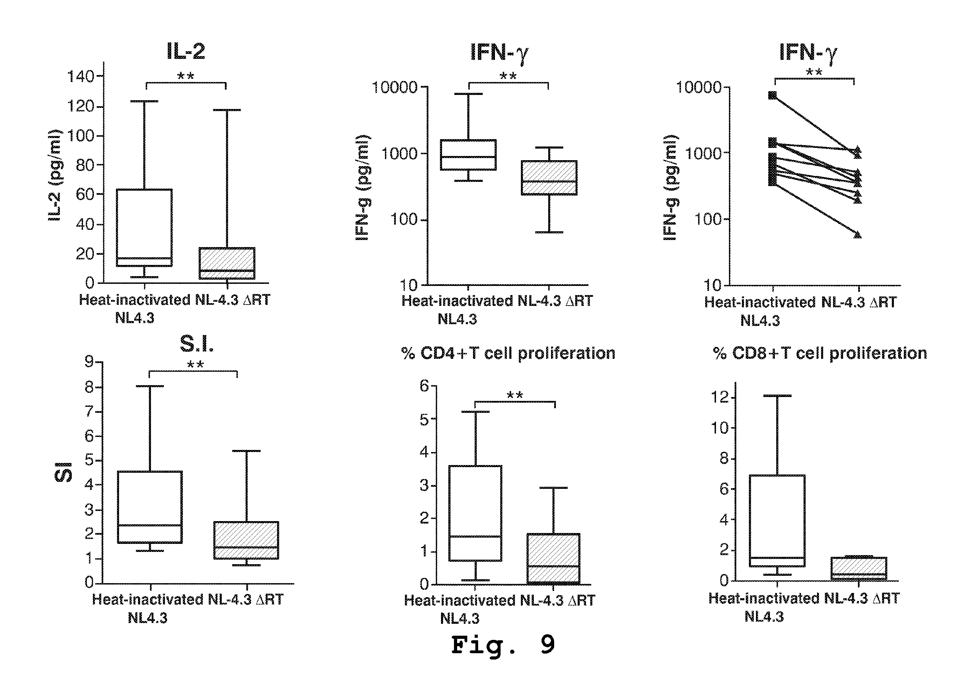

Samples of approximately 20-30 asymptomatic chronic HIV+ patients with a high CD4 number (>500/mm³) were used. An assay for intracellular cytokine detection (ICS) used with different pre-stimulations of the PBMC has fundamentally been used. The pre-stimulations tested were: low doses of recombinant IL-2 (20 U/ml) or a cocktail with CD28 and CD49d (1 µg/ml). No differences have been found between these 2 types of pre-stimulations although the use of the cocktail of antibodies produced greater reproducibility of IFNγ and IL-2 secretion by the CD4 and CD8 cells. The immunogenic antigens assayed were heat-inactivated NL4-3 WT, NL4-3 ΔRT, and BAL and MN (reference strains from laboratory isolates, occasionally known as HIV-BaL and HIV-MN, respectively) inactivated by heat or by means of treatment with 1 mM AT2 (aldrithiol-2) at 4°C overnight (dose corresponding to 20 µg of p24/ml). After stimulating the cells for 18 to 24 hours with the mentioned stimuli, the results obtained were heterogeneous, a marked response being observed in 20-30% of the patients (n≥6), the response induced by the NL4-3 ΔRT being very similar to that obtained by the entirely heat-inactivated virions (Figure 9).

### 3.5 Comparison of the results obtained using the ex vivo model established with PBMC with the MD-DC co-culture model

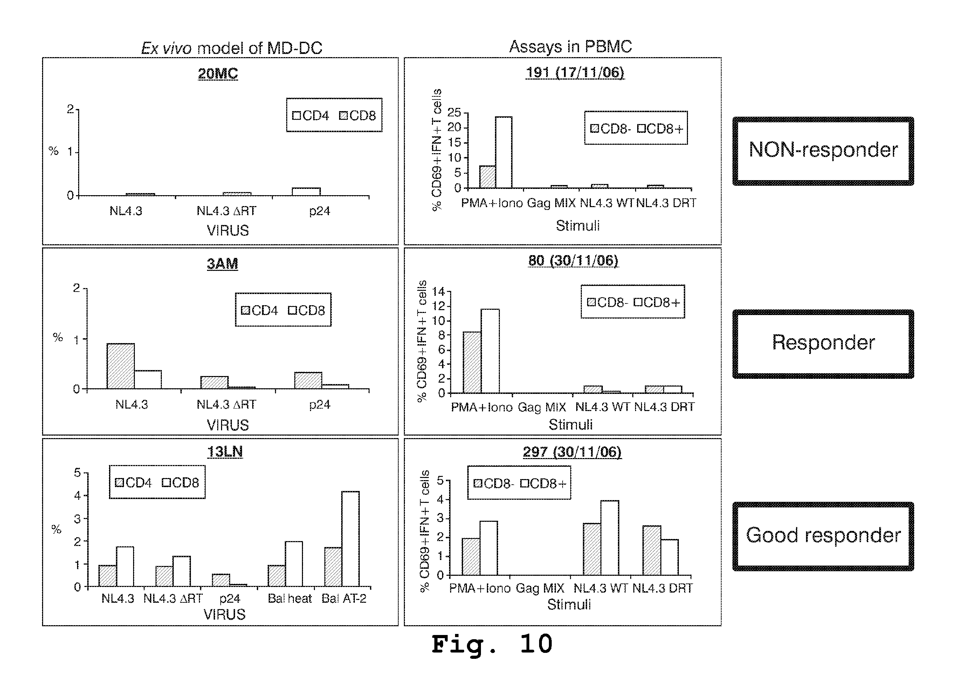

Majority of the *ex vivo* assays with PBMC (ICS) were performed in parallel using both MD-DC and T lymphocytes of the same HIV-infected patients. The preliminary results showed a practically complete parallelism between both assays, the patients responding to the NL4-3 ΔRT virions and other heat-inactivated reference virions can be perfectly discriminated from the non-responding patients. The production of cytokines (IFNγ, IL-2) and the induction of proliferation in CD4 and CD8 cells (Figure 10) were assessed. As observed in Figure 10, there is a parallel grouping of patients depending on their response to the different immunogens between both assays. These data suggest that the ex *vivo* assay with PBMC optimized for the detection of the anti-HIV immune response could allow a rapid *in vitro* assessment of the immunogenic activity of possible future candidates responding to therapeutic vaccines based on MD-DC since the results can be analyzed at 48-72 hours, whereas the results after co-culturing with MD-DC required the prior generation of MD-DC for 7 days and the patient has to be subjected to two blood donations.

### 3.6 Assays with PBMC to detect specific anti-HIV cellular response measured by the number of IFN-γ producing cells (ELISPOT)

Different assays were performed to detect specific anti-HIV cellular response, such as evaluating the immunogenicity of ΔRT against the WT-AT2 (pNL4-3) virion, evaluating the effect of the inactivation by AT2 (aldrithiol-2) (WT-AT2 vs. ΔRT-AT2) and evaluating the efficiency of the tropism of the envelope X4 (ΔRT) vs. R5 (BaL and AC10).

After evaluating the immunogenicity of the ΔRT against WT-AT2 (NL4-3) virion, it was observed that the ΔRT produced response, measured by the number of IFN-γ producing cells (ELISPOT), in a larger number of patients from the analyzed cohort of asymptomatic HIV-1 positive patients. After evaluating the effect of inactivation by AT2 (aldrithiol) (WT-AT2 vs ΔRT-AT2), it was observed that this inactivation process did not influence the immunogenicity of the viral particle. After evaluating the efficacy with respect to the tropism of envelopes X4 (NL4-3) vs. R5 (BaL and AC10) within the ΔRT: the envelopes R5 appeared to be slightly less immunogenic (Table 3).

**Table 3**

| Immune response screening of HIV-1-positive patients. Assessment of the immune response after stimulation with different virions by means of the IFN-γ production (ELISPOT) | |
|---|---|
| PATIENTS /Virions | *Mean SFC* |
| 13 of 59 correspond to WT-AT2 *22%* | *186* |
| 24 of 47 correspond to ΔRT *49%* (X4) | *324* |
| 31 of 47 correspond to ΔRT- *63%* AT2 | *596* |
| 21 of 41 correspond to *51%* ΔRT/AC10 (R5) | *286* |
| 22 of 41 correspond to *54*% ΔRT/BaL (R5) | *315* |

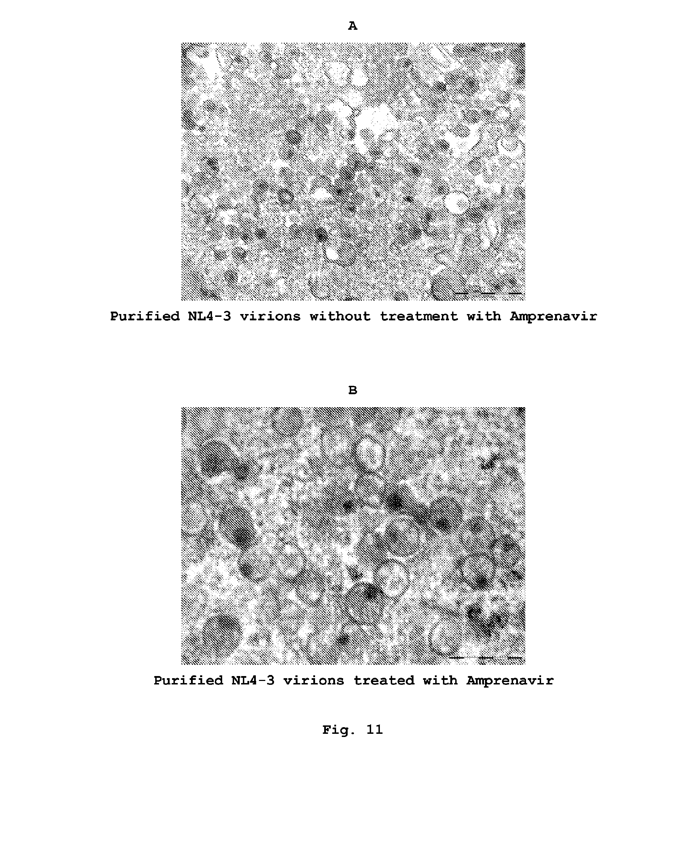

Finally, the immunogenicity of ΔRT was evaluated compared to the WT-AT2 (NL4-3) virion which had been treated previously in their production with a protease inhibitor (Amprenavir, 50 µM APV), WT/APV-AT2. Consequently, treatment with the inhibitor caused the production of immature virions. Said immature particles are shown in Figure 11 by means of electron microscopy. After evaluating the immunogenicity of ΔRT compared to the WT/APV-AT2 virion, it was observed that WT/APV-AT2 produced a response, measured by the number of IFN-γ producing cells (ELISPOT), higher than its untreated homolog, 75% responding patients being obtained in a population of 12 patients.

The autologous chimeras were evaluated as a way of enhancing the response. After evaluating the immunogenicity of ΔRT/Δgag ("patient") virions in patients in whom there was material for amplifying, either DNA or RNA, compared to the different non-chimeric virions described previously, it was observed that the response intensified up to 49%. The heterologous responses observed in other individuals were very heterogeneous. In the responding patients, the use of autologous Gag (for example, patient 596) involved an increase of 33% in the number of SFC with respect to its ΔRT analog. In patients categorized as non-responding patients who have not responded to any of the viral constructs, they only responded to the autologous Gag at its maximum concentration, concentrations greater than 600 ng of p24 antigen (Table 4).

**Table 4**

| Evaluation of autologous chimeras in two patients | | | | |
|---|---|---|---|---|
| *Patient* | WT-AT2 | ΔRT | Gag 70 | Gag 596 |
| 70.ICS | 5 | 15 | 20 | * |
| 596.8 | 55 | 255 | * | 380 |

### EXAMPLE 4

### Immunization model of a patient

In each of the patients who are going to receive vaccinations, i.e., monocyte-derived dendritic cells (MD-DC) pulsed with the infectious HIV-1 immunogen (non-replicative infectious virion), the first step is the isolation and large scale production of HIV-1 by means of transfecting the vector into cells under GMP conditions. Said non-replicative infectious virion comprises a viral genome containing a mutation in the *pol* gene such that the RT protein is non-functional and, in addition, the sequence of another gene different from pol gene substituted with the sequence of the same gene originating from the HIV of the patient. The virus obtained is concentrated and kept at -80°C until it is needed to pulse the MD-DCs of the same patient.

The MD-DCs are freshly prepared from the PBMC monocytes of a 150 ml venous blood sample which is drawn one week prior to each immunization and is immediately processed. After 5 days of culturing the monocytes to generate immature MD-DCs, they are pulsed with the non-replicative infective immunogen for 2 additional culture days, adding thereto a cocktail of MD-DC maturation-inducing cytokines. These will then be washed and administered immediately to the patient through sub-dermal injection close to the axillae (F. Garcia, et al., JIC 2011, 203:473-478).

## Claims

1. A viral vector comprising a human immunodeficiency virus (HIV) genome containing a mutation in the *pol* gene, wherein said mutation causes the virus produced solely from said viral vector to be a non-replicative virus.

2. The viral vector according to claim 1, wherein said mutation in the pol gene comprises the complete or partial deletion of said gene.

3. The viral vector according to claim 1, wherein said mutation in the *pol* gene gives rise to a non-functional RT protein.

4. The viral vector according to any of the preceding claims, wherein said genome further comprises a second mutation in a gene different from the *pol* gene, wherein said second mutation comprises the deletion of a region comprising all or part of said gene different from the *pol* gene.

5. The viral vector according to claim 4, wherein said gene different from the *pol* gene is selected from the group consisting of the *env, gag, nef, tat, rev, vif, vpu* and *vpr* genes.

6. The viral vector according to any of claims 4 or 5, further comprising a marker gene, wherein said marker gene is located at the site occupied by the region deleted by means of said second mutation.

7. The viral vector according to any of claims 4 or 5, further comprising a sequence homologous to that of the region deleted by means of said second mutation, originating from a second HIV genome, wherein the genome of said second HIV is different from the HIV genome containing the deleted region.

8. The viral vector according to claim 7, wherein said second HIV originates from an HIV-infected subject.

9. A non-replicative virion comprising the viral vector according to any of the preceding claims.

10. A method for obtaining an activated immune cell comprising the steps:
a) contacting an immune cell with a non-replicative virion according to claim 9,
b) maintaining the mixture obtained in step a) in conditions suitable for infecting said immune cells with the virion and for processing the antigens originating from the non-replicative virion, and
c) optionally, recovering the activated immune cells.

11. An *in vitro* method for obtaining a customized HIV vaccine for an HIV-infected subject, wherein said vaccine comprises activated immune cells, which comprises:
a) contacting immune cells *in vitro* with a non-replicative virion comprising a viral vector according to claim 7 in which the genome of said second HIV originates from an HIV of said HIV-infected subject;
b) maintaining the mixture obtained in step a) in conditions suitable for infecting said cells with the virion and for processing the antigens originating from the non-replicative virion;
c) detecting and quantifying the activation of the immune cells activated according to steps a) and b);
d) comparing the activation of said immune cells activated according to steps a) and b) with the activation of control immune cells,
e) selecting the virions which cause a greater activation of the immune cells activated according to steps a) and b) than the activation of the control immune cells,
f) infecting immune cells of said HIV-infected subject with the non-replicative virions selected in step e), and
g) recovering the immune cells activated according to step f).

12. An *in vitro* method for obtaining a customized HIV vaccine for an HIV-infected subject, wherein said vaccine comprises activated immune cells, which comprises:
a) contacting immune cells originating from said HIV-infected subject *in vitro* with a non-replicative virion comprising a viral vector according to claim 7 wherein the genome of said second HIV originates from an HIV of said HIV-infected subject;
b) maintaining the mixture obtained in step a) in conditions suitable for infecting said immune cells with the virion and for processing the antigens originating from the non-replicative virion; and
c) recovering the immune cells activated according to step b).

13. The method according to any of claims 10 to 12, wherein said immune cell is selected from a lymphocyte, a peripheral blood mononuclear cell and a monocyte-derived dendritic cell.

14. The method according to claim 11 or 12, further comprising the step of subjecting the activated immune cells to a treatment to ensure the safety of said activated immune cells.

15. A cell obtainable according to the method of any of claims 10 to 12.

16. Use of the non-replicative virion according to claim 9 or of the cell according to claim 15 in medicine.

17. Use of the non-replicative virion according to claim 9 or of the cell according to claim 15 in the preparation of an HIV vaccine.

18. Use of the non-replicative virion according to claim 9 or of the cell according to claim 15 in the preparation of a pharmaceutical composition for treating AIDS.
